# FASCICULE DE BREVET EUROPEEN

(11) **EP 3 359 538 B1**
(45) Date de publication et mention de la délivrance du brevet: **25.03.2020**
(21) Numéro de dépôt: 16778417.2
(22) Date de dépôt: 10.10.2016
(51) Int. Cl.: C07D 471/04, A61K 31/498, A61K 31/4985, A61P 25/16, A61P 25/28

(54) **DERIVÉS DE 1,4,8-TRIAZAPHÉNANTHRÈNE POUR LE TRAITEMENT DE MALADIES NEURODÉGÉNÉRATIVES**
1,4,8-TRIAZAPHENANTHREN-DERIVATE ZUR BEHANDLUNG VON EURODEGENERATIVEN ERKRANKUNGEN
1,4,8-TRIAZAPHENANTHRENE DERIVATIVES FOR THE TREATMENT OF NEURODEGENERATIVE DISORDERS

(30) Priorité: 09.10.2015 FR 1559639
(43) Date de publication de la demande: 15.08.2018
(73) Titulaire: Institut du Cerveau et de la Moelle Epiniere, 75013 Paris (FR); Centre National de la Recherche Scientifique (CNRS), 75016 Paris (FR); Sorbonne Université, 75006 Paris (FR); Assistance Publique - Hôpitaux de Paris, 75004 Paris (FR); Institut National de la Santé et de la Recherche Médicale (INSERM), 75013 Paris (FR); Université Paris-Saclay, 91190 Saint-Aubin (FR)
(72) Inventeur: FIGADERE, Bruno, 91530 Saint Cheron (FR); FERRIE, Laurent, 91120 Palaiseau (FR); LE DOUARON, Gael, 91370 Verrieres Le Buisson (FR); RAISMAN-VOZARI, Rita, 75013 Paris (FR); MICHEL, Patrick, 75004 Paris (FR); SEPULVEDA, Julia, 75005 Paris (FR)
(74) Mandataire: Regimbeau
(86) Numéro de dépôt international: PCT/EP2016/074235
(87) Numéro de publication internationale: WO 2017/060530

(56) Documents cités:
- WO-A1-2012/131080
- LINSKER ET AL.: "Pyridoquinoxalines", JOURNAL OF THE AMERICAN CHEMICAL SOCIETY, vol. 68, 1 janvier 1946 (1946-01-01), pages 874-876, XP002752955,
- SUGURU KONDO ET AL: "Formation of Pyrido(3,2-f)quinoxalines by Reaction of 6-Amino-2,3-dimetylquinoxaline with Aldehydes", CHEMICAL AND PHARMACEUTICAL BULLETIN, PHARMACEUTICAL SOCIETY OF JAPAN, JP, vol. 45, no. 4, 1 janvier 1997 (1997-01-01), pages 722-724, XP009187946, ISSN: 0009-2363
- AKIHIRO OHTA ET AL: "Photocyclization of Styrylpyrazines", CHEMICAL AND PHARMACEUTICAL BULLETIN, PHARMACEUTICAL SOCIETY OF JAPAN, JP, vol. 27, no. 11, 1 janvier 1979 (1979-01-01), pages 2596-2600, XP009187945, ISSN: 0009-2363
- HUISGEN ET AL.: "Beiträge und Deutungsversuche zur reaktionsweise aromatischer Bicyclen", JUSTUS LIEBIGS ANNALENE DER CHEMIE, vol. 559, 1 janvier 1948 (1948-01-01), pages 101-152, XP002752956,

## Description

La présente invention concerne des dérivés 1,4,8-triazaphénanthrène, ainsi que leurs procédés de préparation, les compositions pharmaceutiques les comprenant et leur utilisation comme médicament, notamment dans le traitement de maladies neurodégénératives.

De plus en plus de personnes souffrent de maladies neurodégénératives telles que la maladie d'Alzheimer ou la maladie de Parkinson, en raison de l'allongement de l'espérance de vie.

Une maladie qui affecte le fonctionnement du système nerveux, et en particulier du cerveau, et de façon progressive, est appelée maladie neurodégénérative. Elle peut évoluer plus ou moins rapidement (quelques semaines à plusieurs années), et souvent de façon irréversible. Ainsi, le fonctionnement des cellules nerveuses, en particulier les neurones, se trouve altéré, ce qui peut conduire à leur mort cellulaire. Selon la région du système nerveux atteint par la maladie, différents neurones, et donc différentes fonctions, pourront être affectés comme la motricité, le langage, la mémoire, la perception, l'humeur ou encore la cognition. Parmi les maladies neurodégénératives les plus fréquemment rencontrées, on peut citer en particulier la maladie d'Alzheimer et la maladie de Parkinson.

La maladie d'Alzheimer est une maladie du tissu cérébral entraînant la perte progressive et irréversible des fonctions cognitives, et touche environ 24 millions de personnes dans le monde entier. Le premier symptôme est la perte du souvenir des événements récents (amnésie), puis les déficits cognitifs s'étendent aux domaines du langage (aphasie), de l'organisation des mouvements (apraxie), de la reconnaissance visuelle (agnosie) et des fonctions exécutives (telles que la prise de décision et la planification).

La maladie de Parkinson affecte également le système nerveux central mais provoque des troubles moteurs d'évolution progressive et irréversible, avec notamment des déficits de l'organisation des mouvements et des tremblements du corps.

Aujourd'hui, les médicaments prescrits pour ces deux maladies sont symptomatiques et contribuent à retarder l'évolution de la maladie ; aucun ne permet de guérir la maladie, ni même d'arrêter son évolution, d'où la nécessité de trouver de nouvelles entités chimiques plus actives pour le traitement de ces maladies neurodégénératives.

Les inventeurs de la présente invention ont déjà mis en évidence le potentiel de composés hybrides possédant un noyau amino-quinoxaline dans le traitement de maladies neurodégénératives (WO 2012/131080).

Les inventeurs ont découvert de manière surprenante que les dérivés 1,4,8-triazaphénanthrènes possédaient également une activité neuroprotectrice et étaient capables de passer la barrière hémato-encéphalique.

La présente invention a donc pour objet un composé de formule (I) suivante : 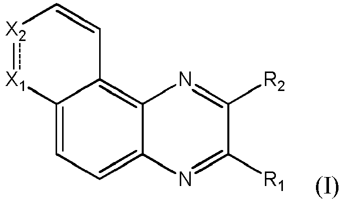 ou un sel et/ou solvate pharmaceutiquement acceptable de celui-ci, un stéréoisomère, ou un mélange de stéréoisomères en toutes proportions, en particulier un mélange d'énantiomères, et notamment un mélange racémique, dans laquelle :
- -̅-̅-̅-̅-̅-̅ représente une liaison simple ou double, de préférence une liaison double,
- X₁ représente :
   ▪ NR₁ₐ lorsque -̅-̅-̅-̅-̅-̅ représente une liaison simple, et
   ▪ N lorsque -̅-̅-̅-̅-̅-̅ représente une liaison double,
- X₂ représente :
   ▪ CR₂ₐR_{2b} lorsque -̅-̅-̅-̅-̅-̅ représente une liaison simple, et
   ▪ CR_{2c} lorsque -̅-̅-̅-̅-̅-̅ représente une liaison double,
- R₁ et R₂ représentent chacun, indépendemment l'un de l'autre, un atome d'hydrogène ; un atome d'halogène tel qu'un atome de chlore, brome ou fluor ; une chaîne hydrocarbonée saturée ou insaturée, linéaire ou ramifiée, comportant de 1 à 10, de préférence 1 à 6 atomes de carbone ; un aryle éventuellement substitué ; ou un hétéroaryle éventuellement substitué,
- R₁ₐ et R_{2c} représentent chacun, indépendemment l'un de l'autre, un atome d'hydrogène ou un groupe (C₁-C₆)alkyle, et
- R₂ₐ et R_{2b} représentent chacun, indépendemment l'un de l'autre, un groupe (C₁-C₆)alkyle.

Le composé de formule (I) pourra se présenter sous la forme d'un ou plusieurs stéréoisomères plus particulièrement lorsque X₁ = CR₂ₐR_{2b} avec R₂ₐ et R_{2b} représentant deux groupes différents et/ou lorsque au moins un groupe parmi R₁, R₂, R₁ₐ, R₂ₐ, R_{2b} et R_{2c} est un groupe chiral.

Selon un mode de réalisation particulier, le composé de formule (I) n'est pas :
- la pyrido[3,2-f]quinoxaline,
- la 2,3-diméthyl-pyrido[3,2-f]quinoxaline,
- la 2,3,8-triméthyl-pyrido[3,2-f]quinoxaline, et
- la 2,3-diphényl-pyrido[3,2-f]quinoxaline,
composés décrits dans la littérature (Linsker et al. J. Am. Chem. Soc. 1946, 68, 874-876 ; Kondo et al. Chem. Pharm. Bull. 1997, 45(4), 722-724 ; Ohta et al. Chem. Pharm. Bull. 1979, 27(11), 2596-2600 ; et Huisgen Justus Liebigs Annalene der Chemie 1948, 559, 101-152), dans des articles de synthèse chimique, aucune activité biologique n'étant reportée pour ces composés.

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

Par « sel pharmaceutiquement acceptable » d'un composé, on entend désigner la présente invention des sels qui sont pharmaceutiquement acceptables, comme défini ici, qui possèdent l'activité pharmacologique souhaitée du composé parent. De tels sels comprennent :
(1) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; et
(2) les sels formés lorsqu'un proton acide présent dans le composé parent soit est remplacé par un ion métallique, par exemple un ion de métal alcalin (Na⁺, K⁺ ou Li⁺ par exemple), un ion de métal alcalino-terreux (comme Ca²⁺ ou Mg²⁺) ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Des solvates acceptables pour l'utilisation pharmaceutique des composés selon la présente invention incluent des solvates conventionnels tels que ceux formés, durant la dernière étape du procédé de préparation des composés selon l'invention, avec le(s) solvant(s) de réaction. A titre d'exemple, il peut être fait mention des solvates formés avec l'eau (appelés communément hydrates) ou avec l'éthanol.

Par groupement « (C₁-C₆)alkyle », on entend, au sens de la présente invention, une chaîne hydrocarbonée saturée, linéaire ou ramifiée, comportant 1 à 6, de préférence 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Par groupement « (C₁-C₆)alcoxy », on entend, au sens de la présente invention, un groupe (C₁-C₆)alkyle tel que défini ci-dessus, lié au reste de la molécule par l'intermédiaire d'un atome d'oxygène. A titre d'exemple, on peut citer les groupes méthoxy, éthoxy, propoxy, isopropoxy, butoxy ou encore *tert*-butoxy.

Par groupement « (C₂-C₆)alcynyle », on entend, au sens de la présente invention, une chaîne hydrocarbonée, linéaire ou ramifiée, comportant au moins une triple liaison et comportant 2 à 6 atomes de carbone. Le groupe (C₂-C₆)alcynyle comportera avantageusement une et une seule triple liaison. A titre d'exemple, on peut citer les groupes éthynyle ou propynyle.

Par groupement « aryle », on entend, au sens de la présente invention, un groupement hydrocarboné aromatique, comportant de préférence de 6 à 10 atomes de carbone et comprenant un ou plusieurs cycles accolés. il s'agira avantageusement d'un groupement phényle ou naphtyle.

Lorsque le groupement aryle est substitué, il pourra avantageusement être substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle.

Par « hétéroaryle », on entend, au sens de la présente invention, un groupe aromatique comprenant un ou plusieurs, notamment 1 ou 2, cycles hydrocarbonés accolés, dans lequel un ou plusieurs atomes de carbone, avantageusement 1 à 4 et encore plus avantageusement 1 ou 2, sont chacun remplacés par un hétéroatome choisis parmi les atomes de soufre, d'azote et d'oxygène et dans lequel chaque cycle comprend avantageusement 5 à 7 chaînons, de préférence 5 ou 6 chaînons. Avantageusement, il s'agira d'un groupe aromatique comprenant 1 ou 2 cycles hydrocarbonés accolés, chaque cycle étant de 5 ou 6 chaînons, dans lequel 1 ou 2 atomes de carbone sont chacun remplacés par un hétéroatome choisis parmi les atomes de soufre, d'azote et d'oxygène, de préférence choisis parmi les atomes d'azote et d'oxygène, tel que l'azote.

Des exemples de groupes hétéroaryle sont les groupes furyle, thiényle, pyrrolyle, isoxazolyle, thiazolyle, isothiazolyle, imidazolyle, pyrazolyle, oxadiazolyle, thiadiazolyle, triazolyle, tétrazolyle, pyridyle, pyridazinyle, pyrimidinyle, pyrazinyle, triazinyle, quinoxalyle ou encore indyle. Il s'agira notamment d'un groupe pyridyle, quinoxalyle ou quinolyle, en particulier pyridyle ou quinolyle.

Lorsque le groupement hétéroaryle est substitué, il pourra avantageusement être substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle.

Par « atome d'halogène », on entend, au sens de la présente invention, les atomes de fluor, de chlore, de brome et d'iode, de préférence de fluor, de chlore et de brome.

Par « insaturée », on entend, au sens de la présente invention, que la chaîne hydrocarbonée peut comportée une ou plusieurs insaturation(s), avantageusement une.

Par « insaturation », on entend, au sens de la présente invention, une double ou une triple liaison carbone-carbone (C=C ou C=C).

Par « stéréoisomère », on entend, au sens de la présente invention, un isomère géométrique ou un isomère optique.

Les isomères géométriques résultent de la position différente des substituants sur une double liaison qui peut avoir alors une configuration Z ou E.

Les isomères optiques résultent notamment de la position différente dans l'espace des substituants sur un atome de carbone comprenant 4 substituants différents. Cet atome de carbone constitue alors un centre chiral ou asymétrique. Les isomères optiques comprennent les diastéréoisomères et les énantiomères. Les isomères optiques qui sont des images l'un de l'autre dans un miroir mais non superposables sont désignés par « énantiomères ». Les isomères optiques qui ne sont ni superposables, ni images l'un de l'autre dans un miroir sont désignés par « diastéréoisomères ».

Un mélange contenant des quantités égales de deux formes énantiomères individuelles de chiralité opposée est désigné par « mélange racémique ».

Par « groupe chiral », on entend, au sens de la présente invention, un groupe qui n'est pas superposable à son image dans un miroir. Un tel groupe chiral pourra comprendre en particulier un atome de carbone chiral, c'est-à-dire un atome de carbone substitué par quatre substituants (incluant l'hydrogène) différents.

Selon un mode de réalisation préféré, -̅-̅-̅-̅-̅-̅ représente une liaison double. En conséquence, X₁ représente N et X₂ représente un groupe CR_{2c} dans ce mode de réalisation préféré. R_{2c} représentera avantageusement un atome d'hydrogène. Un composé selon la présente invention sera ainsi avantageusement un composé de formule (la) suivante : 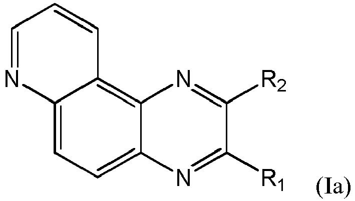 ou un sel et/ou solvate pharmaceutiquement acceptable de celui-ci, un stéréoisomère, ou un mélange de stéréoisomères en toutes proportions, en particulier un mélange d'énantiomères, et notamment un mélange racémique,
dans laquelle R₁ et R₂ sont tels que définis ci-dessus ou ci-dessous.

Dans la définition de R₁ et R₂, le terme « chaîne hydrocarbonée saturée ou insaturée, linéaire ou ramifiée, comportant de 1 à 10, de préférence 1 à 6 atomes de carbone» représentera plus particulièrement :
- une chaîne hydrocarbonée saturée, linéaire ou ramifiée, comportant de 1 à 10, de préférence 1 à 6 atomes de carbone, et plus particulièrement un groupe (C₁-C₆)alkyle tel que défini ci-dessus, ou
- une chaîne hydrocarbonée insaturée, linéaire ou ramifiée, comportant de 1 à 10, de préférence 1 à 6 atomes de carbone et comportant au moins une triple liaison, et avantageusement comportant une et une seule triple liaison comme insaturation, et plus particulièrement un groupe (C₂-C₆)alkynyle tel que défini ci-dessus.

Avantageusement, R₁ et R₂ représentent chacun, indépendemment l'un de l'autre :
- un atome d'hydrogène,
- un atome d'halogène tel que le chlore, le fluor et le brome,
- une chaîne hydrocarbonée saturée ou insaturée, linéaire ou ramifiée, comportant de 1 à 10, de préférence 1 à 6 atomes de carbone,
- un aryle éventuellement substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH, ou
- un hétéroaryle éventuellement substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH.

En particulier, R₁ et R₂ pourront représenter chacun, indépendemment l'un de l'autre :
- un atome d'hydrogène,
- un atome d'halogène tel que le chlore, le fluor et le brome,
- un (C₁-C₆)alkyle,
- un (C₂-C₆)alkynyle, comportant avantageusement une et une seule triple liaison,
- un aryle éventuellement substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH, ou
- un hétéroaryle éventuellement substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH.

De péférence, R₁ et R₂ représenteront chacun, indépendemment l'un de l'autre :
- un atome d'hydrogène,
- un (C₁-C₆)alkyle,
- un (C₂-C₆)alkynyle, comportant avantageusement une et une seule triple liaison,
- un aryle éventuellement substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH, ou
- un hétéroaryle éventuellement substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH.

Avantageusement, R₁ et R₂ représentent chacun, indépendemment l'un de l'autre, un atome d'hydrogène ; un groupe (C₁-C₆)alkyle ; ou un groupe aryle ou hétéroaryle éventuellement substitué, notamment par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH.

En particulier, R₁ et R₂ pourront représenter chacun, indépendemment l'un de l'autre, un atome d'hydrogène ; un groupe (C₁-C₆)alkyle ; ou un groupe phényle, naphtyle, pyridyle, quinoxalyle ou quinolyle, notamment phényle, naphtyle, pyridyle ou quinolyle, éventuellement substitué, notamment par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH.

Selon un mode de réalisation particulier de l'invention, R₁ représentera un atome d'hydrogène ou un groupe (C₁-C₆)alkyle tel que méthyle, notamment un atome d'hydrogène.

Selon un autre mode de réalisation particulier de l'invention, R₂ représentera un groupe aryle ou hétéroaryle, de préférence aryle, éventuellement substitué, notamment par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH. R₂ pourra représenter en particulier un groupe phényle, naphtyle, pyridyle, quinoxalyle ou quinolyle, notamment phényle, naphtyle, pyridyle ou quinolyle, de préférence phényle, éventuellement substitué, notamment par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH.

Selon un mode de réalisation préféré de l'invention, R₁ représentera un atome d'hydrogène ou un groupe (C₁-C₆)alkyle tel que méthyle, notamment un atome d'hydrogène, et R₂ représentera un groupe un groupe aryle ou hétéroaryle, de préférence aryle, éventuellement substitué, notamment par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH. R₂ pourra représenter en particulier un groupe phényle, naphtyle, pyridyle, quinoxalyle ou quinolyle, notamment phényle, naphtyle, pyridyle ou quinolyle, de préférence phényle, éventuellement substitué, notamment par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH.

Dans les définitions de R₁ et R₂ ci-dessus, le groupe (C₁-C₆)alkyle pourra être plus particulièrement un groupe méthyle, *n*-butyle, *s*-butyle, *t*-butyle ou *n*-hexyle, tel que méthyle ; le groupe (C₂-C₆)alkynyle pourra être plus particulièrement un groupe éthynyle ; le groupe aryle éventuellement substitué pourra être plus particulièrement un groupe phényle ou naphtyle, de préférence phényle, éventuellement substitué, tel qu'un groupe phényle, 1-naphthyle, 2-naphthyle, *m*-hydroxyphényle, *m*-méthoxyphényle, *p*-méthoxyphényle, *p-*chlorophényle, *p*-fluorophényle, *p*-méthylphényle, 3,4-diméthoxyphényle, 3,4,5-triméthoxyphényle, 3,4-dichlorophényle, ou biphényle ; le groupe hétéroaryle éventuellement substitué pourra être plus particulièrement un groupe pyridyle, quinoxalyle ou quinolyle, notamment pyridyle ou quinolyle, éventuelle substitué et notamment non substitué, tel qu'un groupe 3-pyridyle ou 3-quinolyle.

Les composés de formule (I) pourront notamment être choisis parmi les composés suivants :

| | | | |
|---|---|---|---|
| **Iac** | 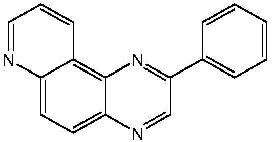 | **Iaf** | 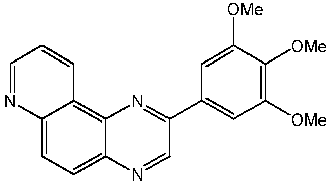 |
| **Iaa** | 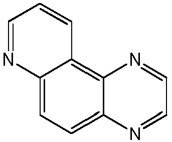 | **Iag** | 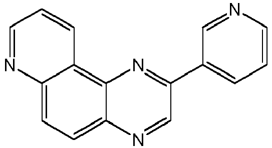 |
| **Iad** | 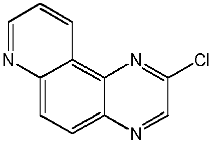 | **Iam** | 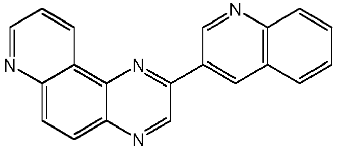 |
| **Iae** | 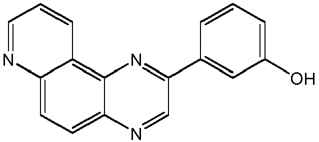 | **Iah** | 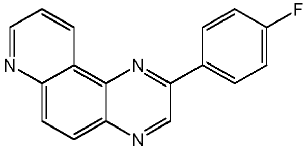 |
| **Iai** | 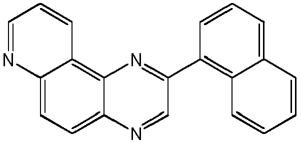 | **Iaj** | 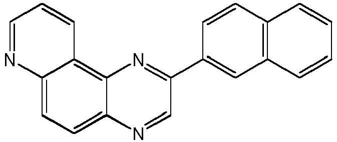 |
| **Iak** | 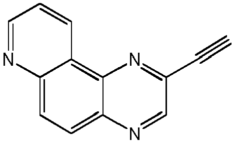 | **Ibn** | 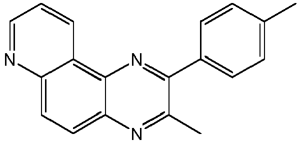 |
| **Ibl** | 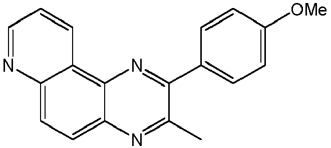 | **Ibf** | 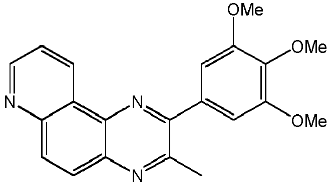 |
| **Ibo** | 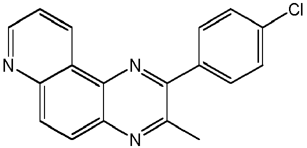 | **Ibp** | 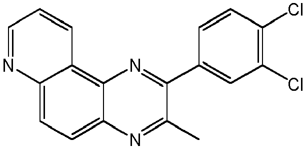 |
| **Ibh** | 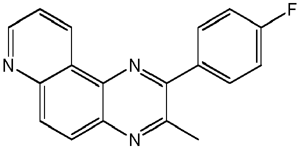 | **Ibq** | 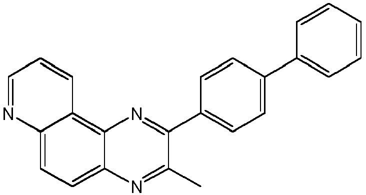 |
| **Ibj** | 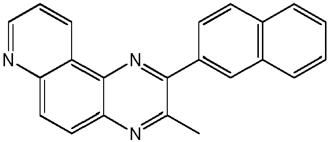 | **Xbc** | 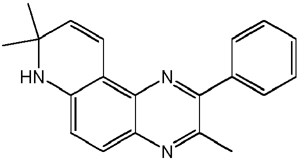 |

et leurs sels et/ou solvates pharmaceutiquement acceptables.

La présente invention a également pour objet un composé de formule (I) tel que défini ci-dessus, pour son utilisation en tant que médicament, notamment en tant que médicament neurotrophique ou neuroprotecteur, et plus particulièrement pour son utilisation dans le traitement ou la prévention d'une maladie neurodégénérative.

La présente invention concerne également l'utilisation d'un composé de formule (I) tel que défini ci-dessus pour la fabrication d'un médicament, notamment d'un médicament neurotrophique ou neuroprotecteur, et plus particulièrement pour la fabrication d'un médicament destiné au traitement ou à la prévention d'une maladie neurodégénérative.

La présente invention concerne également une méthode de traitement ou de prévention d'une maladie neurodégénérative comprenant l'administration d'une quantité efficace d'un composé de formule (I) tel que défini ci-dessus à un patient en ayant besoin.

La maladie neurodégénérative pourra être plus particulièrement la maladie d'Alzheimer, la maladie de Parkinson, la sclérose en plaques ou la sclérose latérale amyotrophique (SLA) encore appelée maladie de Charcot, et en particulier la maladie de Parkinson.

La présente invention a également pour objet une composition pharmaceutique comprenant au moins un composé de formule (I) tel que défini ci-dessus et un véhicule pharmaceutiquement acceptable, à l'exclusion d'une solution de pyrido[3,2-f]quinoxaline dans l'eau.

Par « composition pharmaceutique », on entend, au sens de la présente invention, une composition possédant des propriétés curatives ou préventives à l'égard de maladies, telles les maladies neurodégénératives dans le cas d'espèce, destinée à être administrée à un animal, notamment un mammifère tel que l'homme.

Les compositions pharmaceutiques selon l'invention peuvent être formulées pour une administration parentérale (par exemple sous-cutanée, intra-péritonéale, intramusculaire, intraveineuse, intracranienne, intrathécale, etc.), orale, sublinguale, transdermique, locale ou rectale, destinée aux mammifères, y compris l'homme. La posologie varie selon le traitement et selon l'affection en cause.

Dans les compositions pharmaceutiques de la présente invention, l'ingrédient actif peut être administré sous formes unitaires d'administration, en mélange avec des supports pharmaceutiques classiques, aux animaux ou aux êtres humains.

Les formes unitaires d'administration par voie orale appropriées comprennent les comprimés, les gélules, les poudres, les granules et les solutions ou suspensions orales, et les formes d'administration parentérale, notamment intra-péritonéale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs de goût ou des édulcorants.

Pour une administration parentérale, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

Les composés de l'invention peuvent être utilisés à des doses comprises entre 0,01 mg et 1000 mg par jour, donnés en une seule dose une fois par jour ou administrés en plusieurs doses tout au long de la journée, par exemple deux fois par jour en doses égales. La dose administrée par jour est avantageusement comprise entre 5 mg et 500 mg, encore plus avantageusement entre 10 mg et 200 mg. Il peut être nécessaire d'utiliser des doses sortant de ces gammes ce dont l'homme du métier pourra se rendre compte lui-même.

Selon un mode de réalisation particulier, la composition pharmaceutique telle que définie ci-dessus pourra comprendre en outre un autre principe actif, utile notamment dans le traitement ou la prévention de maladies neurodégénératives, et avantageusement choisi parmi des inhibiteurs d'acétylcholinestérase tels que le donépézil, la galanthamine, la rivastigmine, la mémantine et la tacrine ; des inhibiteurs de monoamines oxydase tels que la sélégiline ou rasagiline ; des inhibiteurs de catécholamines O-méthyltransférase tels que l'entacapone ; des inhibiteurs glutamatergiques tels que l'amantadine et le baclofène ; des agonistes cholinergiques tels que la sabcoméline ; des agonistes dopaminergiques tels que le pergolide, le cabergoline, le ropirinole et le pramipexole ; des analogues ou précurseurs de neuromédiateurs tels que la L-3,4-dihydroxyphénylalanine ; et des anticholinergiques tels que le trihexyphénidyl et la tropatépine.

La présente invention a également pour objet une composition pharmaceutique selon l'invention pour son utilisation en tant que médicament neurotrophique ou neuroprotecteur, et plus particulièrement pour son utilisation dans le traitement ou la prévention d'une maladie neurodégénérative, notamment telle que définie précédemment.

La présente invention concerne également l'utilisation d'une composition pharmaceutique selon l'invention pour la fabrication d'un médicament neurotrophique ou neuroprotecteur, et plus particulièrement pour la fabrication d'un médicament destiné au traitement ou à la prévention d'une maladie neurodégénérative, notamment telle que définie précédemment.

La présente invention concerne également une méthode de traitement ou de prévention d'une maladie neurodégénérative, notamment telle que définie précédemment, comprenant l'administration d'une quantité efficace d'une composition pharmaceutique selon l'invention à un patient en ayant besoin.

La présente invention a également pour objet un premier procédé de préparation d'un composé de formule (I) tel que défini ci-dessus comprenant les étapes successives suivantes :
(a1) couplage entre une amino-quinoxaline de formule (II) suivante : 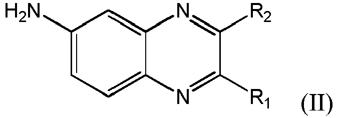 pour laquelle R₁ et R₂ sont tels que définis précédemment,
   avec un halogénure propargylique de formule CH≡C-CHR_{2c}Hal ou CH≡C-CR₂ₐR_{2b}Hal dans laquelle R₂ₐ, R_{2b} et R_{2c} sont tels que définis précédemment et Hal représente un atome d'halogène tel que Cl, Br ou I, notamment Br ou Cl, pour donner un composé de formule (IIIa) ou (IIIb) suivante : 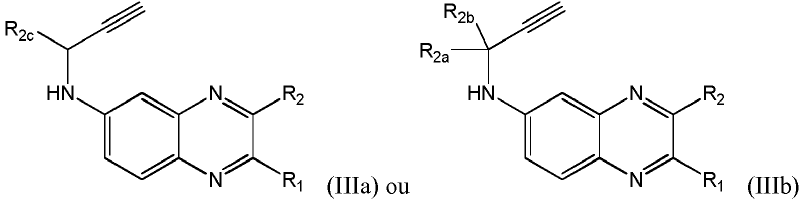 pour laquelle R₁, R₂, R₂ₐ, R_{2b} et R_{2c} sont tels que définis précédemment,
(bl) cycloisomérisation du composé de formule (IIIa) ou (IIIb) obtenu à l'étape précédente et aromatisation lorsque -̅-̅-̅-̅-̅-̅ représente une liaison double pour donner un composé de formule (I), et
(c1) éventuellement salification et/ou solvatation du composé de formule (I) obtenu à l'étape précédente pour donner un sel et/ou solvate pharmaceutiquement acceptable du composé de formule (I).

### Etape (a1) :

Les produits de départ utilisés pour cette étape (aminoquinoxaline de formule (II)) sont disponibles dans le commerce ou peuvent être facilement préparés par des méthodes bien connues de l'homme du métier. Leur synthèse est notamment décrite dans les précédentes demandes internationales WO 2010/007179 et WO 2012/131080. Des procédés de synthèse sont également illustrés dans la partie expérimentale ci-après.

Cette réaction de couplage sera avantageusement réalisée en présence d'une base telle que K₂CO₃ ou K₃PO₄, et notamment un iodure tel que KI, NaI ou nBu₄NI.

Cette réaction peut être avantageusement réalisée dans un solvant tel que le diméthylfomamide (DMF), le dioxane, la NMP (N-méthyl pyrrolidinone), le DMSO (diméthylsulfoxyde), la DMPU (1,3-diméthyl-3,4,5,6-tétrahydro-2(1H)-pyrimidinone), l'HMPA (hexaméthylphosporamide), le DMA (diméthylacétamide) ou un mélange de ceux-ci, et en particulier le DMF, notamment à une température comprise entre 50 et 140°C, notamment d'environ 80°C.

### Etape(b1) :

La réaction de cycloisomérisation sera avantageusement réalisée en présence d'un catalyseur tel qu'un dérivé de cuivre (I) (par ex. CuCl, Cu₂O, CuOTf, CuPF₆ ou CuBF₄) ou encore un sel d'argent (par ex. AgNO₃, Ag₂CO₃, AgF, AgPF₆, AgOTf, AgBF₄ ou CF₃C(O)OAg). Le catalyseur sera plus particulièrement CuCl.

Cette réaction peut être avantageusement réalisée dans un solvant tel que le diméthylsulfoxyde (DMSO), le chloroforme, le toluène, le DMF, le dioxane, la NMP, la DMPU, l'HMPA, le DMA ou un mélange de ceux-ci, et en particulier le DMSO ou le toluène, notamment à une température comprise entre 70 et 160°C, notamment d'environ 120°C.

Cette réaction de cycloisomérisation s'accompagne spontanément d'une aromatisation du système tricyclique lorsque l'étape (bl) est effectuée à partir d'un composé de formule (IIIa) pour donner un dérivé de type 1,4,8-triazaphénanthrène.

### Etape(c1) :

L'étape de salification pourra être réalisée dans des conditions bien connues de l'homme du métier, en présence d'un acide ou d'une base pharmaceutiquement acceptable, notamment tel(le) que défini(e) précédemment.

Lorsque le composé se trouve sous une forme solvatée, cette solvatation a généralement lieu dans la dernière étape du procédé, le solvant de la forme solvatée étant dans ce cas le solvant du milieu réactionnel.

La présente invention a également pour objet un second procédé de préparation d'un composé de formule (I) ci-dessus pour lequel au moins l'un de R₁ et R₂ représente une chaîne hydrocarbonée saturée ou insaturée, linéaire ou ramifiée, comportant de 1 à 10 atomes de carbone ; un aryle éventuellement substitué ; ou un hétéroaryle éventuellement substitué comprenant les étapes successives suivantes :
(a2) couplage d'un composé de formule (I) selon l'invention pour lequel au moins l'un de R₁ et R₂ représente un atome d'halogène, tel que Cl, Br ou I, notamment Cl, avec un dérivé d'acide boronique de formule R₃-B(R₄)₂ ou R₃-BF₃⁻K⁺ pour lequel R₃ représente une chaîne hydrocarbonée saturée ou insaturée, linéaire ou ramifiée, comportant de 1 à 10 atomes de carbone ; un aryle éventuellement substitué ; ou un hétéroaryle éventuellement substitué, et R₄ représente un groupe (C₁-C₆)alkyle, OH ou (C₁-C₆)alcoxy,
   ou avec un dérivé zincique de formule R₃-Zn-Hal pour lequel R₃ est tel que défini ci-dessus et Hal représente un atome d'halogène tel que Cl, Br ou I, notamment Cl, ou avec un dérivé stannane de formule R₃-SnA₁A₂A₃ pour lequel R₃ est tel que défini ci-dessus et A₁, A₂ et A₃, identiques ou différents, représentent chacun un groupe (C₁-C₆)alkyle,
   ou avec un dérivé magnésien de formule R₃-Mg-Hal pour lequel R₃ est tel que défini ci-dessus et Hal représente un atome d'halogène tel que Cl, Br ou I, notamment Br,
   ou avec un dérivé silicié de formule R₃-SiMe₂OH, R₃-SiF₃ ou R₃-Si(OA₁)(OA₂)(OA₃) pour lequel R₃, A₁, A₂ et A₃ sont tels que définis ci-dessus, ou avec un alcyne de formule R'-C=CH pour lequel R' représente un groupe protecteur ou une chaîne hydrocarbonée saturée ou insaturée, linéaire ou ramifiée, comportant de 1 à 8 atomes de carbone,
(b2) lorsque l'étape (a2) a été réalisée avec un alcyne de formule R'-C=CH pour lequel R' représente un groupe protecteur, déprotection du groupe protecteur de la fonction alcyne, et
(c2) éventuellement salification et/ou solvatation du composé de formule (I) obtenu à l'étape précédente pour donner un sel et/ou solvate pharmaceutiquement acceptable du composé de formule (I).

### Etape (a2) :

### Couplage avec un acide boronique de formule R₃-B(R₄)₂ ou R₃-BF₃⁻K⁺ :

Ce couplage pourra être réalisé dans les conditions de la réaction de Suzuki bien connues de l'homme du métier.

Cette réaction de couplage sera ainsi avantageusement réalisée en présence d'un catalyseur au palladium tel que Pd(PPh₃)₄, Pd(OAc)₂, PdCl₂(dppf) ou PdCl₂(PPh₃)₂, notamment PdCl₂(PPh₃)₂.

Elle pourra être réalisée en présence d'une base telle que K₂CO₃, K₃PO₄, Na₂CO₃, Cs₂CO₃, KOH, CsOH ou NaOH, notamment K₂CO₃.

Cette réaction peut être avantageusement réalisée dans un solvant choisi parmi le dioxane, l'eau et leurs mélanges, plus particulièrement dans un mélange eau/dioxane, notamment au reflux.

Le dérivé d'acide boronique utilisé pourra être plus particulièrement un acide boronique de formule R₃-B(OH)₂.

### Couplage avec un dérivé zincique de formule R₃-Zn-Hal :

Ce couplage pourra être réalisé dans les conditions de la réaction de Negishi bien connues de l'homme du métier.

Cette réaction de couplage sera ainsi avantageusement réalisée en présence d'un catalyseur au palladium tel que Pd(PPh₃)₄. Un catalyseur au nickel peut aussi être envisagé.

Cette réaction peut être avantageusement réalisée dans un solvant tel que le tétrahydrofurane (THF).

### Couplage avec un dérivé stannane de formule R₃-SnA₁A₂A₃:

Ce couplage pourra être réalisé dans les conditions de la réaction de Stille bien connues de l'homme du métier.

Cette réaction de couplage sera ainsi avantageusement réalisée en présence d'un catalyseur au palladium tel que Pd(PPh₃)₄. L'ajout de sels de cuivre tel que CuTC, CuDPP, ou CuCl pourra être utilisé pour faciliter la réaction.

A₁, A₂ et A₃ seront avantageusement identiques, et représenteront notamment Me ou Bu, notamment Bu.

### Couplage avec un dérivé magnésien de formule R₃-Mg-Hal :

Ce couplage pourra être réalisé dans les conditions de la réaction de Kumada bien connues de l'homme du métier.

Cette réaction de couplage sera ainsi avantageusement réalisée en présence d'un catalyseur au palladium tel que Pd(PPh₃)₄ ou PdCl₂(dppf) ou d'un catalyseur au nickel tel que Ni(acac)₂, NiCl₂(dppp), NiCl₂(dppe) ou NiCl₂(dppb).

### Couplage avec un dérivé silicié de formule R₃-SiMe₂OH, R₃-SiF₃ ou R₃-Si(OA₁)(OA₂)(OA₃) :

Ce couplage pourra être réalisé dans les conditions de la réaction de Hiyama bien connues de l'homme du métier.

Cette réaction de couplage sera ainsi avantageusement réalisée en présence d'un catalyseur au palladium tel que PdCl₂, Pd₂dba₃, Pd(OAc)₂, Pd(PPh₃)₄ ou PdCl₂(dppf) ou d'un catalyseur au nickel tel que Ni(acac)₂, NiCl₂(dppp), NiCl₂(dppe), NiCl₂(dppb), NiBr₂.diglyme ou NiCl₂.glyme.

Cette réaction de couplage sera également avantageusement réalisée en présence d'une source de fluorure tel que TBAF (fluorure de tétra-*n*-butylammonium), TASF (difluorotriméthylsilicate de tris(diméthylamino)sulfonium) ou CsF, ou bien d'hydroxyde tel que NaOH ou KOH

A₁, A₂ et A₃ seront avantageusement identiques, et représenteront notamment Me ou Et.

### Couplage avec un alcyne de formule R'-C≡CH:

Ce couplage pourra être réalisé dans les conditions de la réaction de Sonogashira bien connues de l'homme du métier.

Cette réaction de couplage sera ainsi avantageusement réalisée en présence d'un catalyseur au palladium tel que Pd(PPh₃)₄ ou PdCl₂(PPh₃)₂, notamment PdCl₂(PPh₃)₂, et d'un sel de cuivre (I) tel que CuI ou CuBr, notamment CuI.

Elle pourra être réalisée en présence d'une base telle qu'une amine tertiaire ou secondaire, par exemple NHEt₂, NEt₃ ou NEt(*i*Pr)₂, notamment NEt₃.

Cette réaction peut être avantageusement réalisée dans un solvant choisi parmi le THF (tétrahydrofurane), l'acétonitrile, l'acétate d'éthyle et leurs mélanges, plus particulièrement dans du THF, notamment au reflux.

Le couplage de l'étape (a2) sera avantageusement réalisée par une réaction de Suzuki ou de Sonogashira.

### Etape(b2) :

Le groupe protecteur de la fonction alcyne pourra être un groupemnt silylé tel que SiA₄A₅A₆ où A₄, A₅ et A₆ représentent chacun, indépendamment les uns des autres, un groupe (C₁-C₆)alkyle. Il pourra s'agir plus particulièrement d'un groupe triméthylsilyle (TMS).

Il peut être déprotégé dans des conditions bien connues de l'homme du métier, et notamment en présence d'une base telle K₂CO₃ dans le methanol comme solvant ou bien en présence d'une source de fluorure tel que TBAF dans le THF comme solvant. Une telle déprotection pourra être réalisée à température ambiante ou en chauffant à une température pouvant aller jusqu'au reflux du solvant.

Un composé de formule (I) avec au moins l'un de R₁ et R₂ représentant un groupe -C=CH est ainsi obtenu.

### Etape(c2) : voir étape (c1) précédente.

Le composé obtenu par l'un des deux procédés ci-dessus pourra être séparé du milieu réactionnel par des méthodes bien connues de l'homme du métier, comme par exemple par extraction, évaporation du solvant ou encore par précipitation et filtration.

Il pourra également être purifié si nécessaire par des techniques bien connues de l'homme du métier, comme par recristallisation si le composé est cristallin, par distillation, par chromatographie sur colonne sur gel de silice ou alumine ou encore par chromatographie liquide haute performance (HPLC).

La présente invention sera mieux comprise à la lumière des exemples non limitatifs qui suivent.

### EXEMPLES

Les abréviations suivantes ont été utilisées dans cette partie.
- AMP: : Adénosine 3',5'-monophosphate
- dbc-AMP: : Dibutyryl adénosine 3',5'-monophosphate cyclique
- BHE: : Barrière hémato-encéphalique
- DCM: : Dichlorométhane
- DMSO: : Diméthylsulfoxyde
- équiv.: : Equivalent
- ESI: : Ionisation par électrospray
- HPLC: : Chromatographie Liquide Haute Performance
- HPLC-: : Chromatographie Liquide Haute Performance couplée à deux
- MS/MS: spectrométries de masse
- IR: : Infrarouge
- MRM: : Multiple Reaction Monitoring
- MS: : Spectre de masse
- RMN: : Résonance Magnétique Nucléaire
- THF: : Tétrahydrofurane
- UHPLC: : Chromatographie Liquide Ultra Haute Performance
- UV: : Ultraviolet

Les composés selon l'invention ont été nommés sous la forme Ixy (lorsque-̅-̅-̅-̅-̅-̅ représente une liaison simple et R_{2c} = H) ou Xxy (lorsque -̅-̅-̅-̅-̅-̅ représente une liaison double et R₂ₐ = R_{2b} = Me) où :
- le premier suffixe x correspond à R₂,
- le second suffixe y correspond à R₁,
avec pour les suffixes : **a** = H, **b** = Me, c = Ph, **d** = Cl, **e** = *m*-hydroxyphényle, **f** = 3,4,5-triméthoxyphényle, **g** = 3-pyridyle, **h** = *p*-fluorophényle, **i** = 1-naphthyle, **j** = 2-naphthyle, **k** = 1-ethynyle, **l** = *p*-méthoxyphényle, **m** = 3-quinolyle, **n** = *p*-méthylphényle, **o** = *p-*chlorophényle, **p** = 3,4-dichlorophényle, **q** = biphényle.

La même nomenclature a été utilisée pour les intermédiaires de synthèse de formule (II) ou (IIIa) (avec R_{2c} = H) sous la forme IIxy et IIIxy respectivement.

La synthèse des aminoquinoxalines de formule (II) utilisées dans les exemples est soit décrite ci-dessous, soit décrite dans WO 2010/007179 ou WO 2012/131080.

### I. Synthèse des composés selon l'invention

### Exemple 1 : synthèse du composé Ibl

Etape 1 : A une solution de composé **IIbl** (343 mg, 1,29 mmol, 1 equiv.) dans le diméthyformamide anhydre sont ajoutés 178 mg de K₂CO₃ (1,29 mmol, 1 equiv.), 214 mg de KI (1,29 mmol, 1 equiv.) et 0,288 ml de bromure de propargyle (1,7 mmol, 2 equiv., 80% dans le toluène). Le milieu réactionnel est ensuite chauffé à 80°C pendant 24 h. Après refroidissement, le milieu réactionnel est hydrolysé par une solution saturée de K₂CO₃, puis extrait 3 fois avec de l'acétate d'éthyle. Les phases organiques sont rassemblées, lavées avec une solution saturée de NaCl, séchées sur Na₂SO₄, filtrées puis concentrées sous pression réduite. Une purification par chromatographie sur gel de silice dans un mélange cyclohexane : acétate d'éthyle en proportions 7:3 puis 6:4 a permis d'obtenir le composé **IIIbl** (71%, 280,0 mg) ainsi que le composé disubstitué (14%, 64,1 mg). **RMN ¹H (400 MHz, CDCl₃) δ ppm** : 2.24 (t, *J* = 2.4 Hz, 1H), 2.70 (s, 3H), 3.86 (s, 3H), 4.03 (d, *J* = 2.4 Hz, 2H), 4.44 (brs, 1H, NH), 7.02 (d, *J* = 8.7 Hz, 2H), 7.09 (dd, *J* = 8.7, 2.5 Hz, 1H), 7.12 (d, *J* = 2.5 Hz, 1H), 7.60 (dd, *J* = 8.7 Hz, 2H), 7.81 (d, *J* = 8.7 Hz, 1H). **RMN ¹³C (100 MHz, CDCl₃) δ ppm :** 23.9, 33.4, 55.3, 71.8, 80.0, 105.3, 113.8 (2C), 121.4, 128.9, 130.4 (2C), 131.9, 136.1, 142.9, 147.2, 148.1, 154.4, 160.0. **MS (ESI) m/z** : 304.3 ([M+H]⁺, 100). **Masse haute résolution (ESI):** m/z calculé pour [M+H]⁺ C₁₉H₁₈N₃O : 304.1450 ; m/z mesuré : 304.1458. **Pureté (HPLC/UV λ à 260 nm) :** 100 %. Etape 2 : A une solution du composé **IIIbl** (50 mg, 0.166 mmol, 1 equiv.) dans le DMSO mL) est additionné du CuCl (26 mg, 0.183 mmol, 1.1 equiv). Après 8 h à 120°C, le milieu réactionnel est refroidi à température ambiante, puis est additionné NH₃ à 28% (5mL), de CH₂Cl₂ (10 mL) et H₂O (10 mL). La phase aqueuse est extraite 3 fois au CH₂Cl₂, puis phases organiques rassemblés sont lavés 2 fois avec une solution aqueuse de NaCl séchés sur MgSO₄, puis concentrés. Après purification sur silice (85:15 / DCM:AcOEt), le produit **Ibl** est obtenu (30,2 mg, 62%). **RMN ¹H (400 MHz, CDCl₃) δ ppm :** 2.87 (s, 3H), 3.90 (s, 3H), 7.07 (d, *J* = 8.8 Hz, 2H), 7.59 (dd, *J* = 8.0, 4.1 Hz, 1H), 7.75 (d, *J* = 8.8 Hz, 2H), 8.12 (d, *J* = 9.3 Hz, 1H), 8.23 (d, *J* = 9.3 Hz, 1H), 9.03 (brs, 1H), 9.45 (dd, *J* = 8.0 et 0.8 Hz, 1H). **RMN ¹³C (100 MHz, CDCl₃) δ ppm :** 24.4, 55.4, 113.9 (2C), 122.0 (br), 126.4 (br), 129.8, 130.8 (2C), 131.2, 132.1, 132.5, 138.2, 139.7, 148.9 (br), 151.2 (br), 152.4, 152.8, 160.4. **MS (ESI) m/z :** 302.2 ([M+H]⁺, 100). **Masse haute résolution (ESI)** : m/z calculé pour [M+H]⁺ C₁₉H₁₆N₃O : 302.1293 ; m/z mesuré : 302.1296. **Pureté (HPLC/UV λ à 260 nm) :** 100 %.

Les composés suivant ont également été synthétisés selon cette procédure :
**Iaa : RMN ¹H (300 MHz, CDCl₃)** δ 9.47 (ddd, *J* = 8.3, 1.8, 0.5 Hz, 1H), 9.09 (dd, *J* = 4.4, 1.8 Hz, 1H), 8.96 (d, *J* = 2.0 Hz, 1H), 8.94 (d, *J* = 2.0 Hz, 1H), 8.30 (dd, *J* = 9.3, 0.5 Hz, 1H), 8.20 (d, *J* = 9.3 Hz, 1H), 7.68 (dd, *J* = 8.3, 4.4 Hz, 1H).
**Ibn : Rendement:** 61% (30.5 mg).**RMN ¹H (300 MHz, CDCl₃)** δ 9.47 (d, *J* = 7.9 Hz, 1H), 9.04 (d, *J* = 4.4 Hz, 1H), 8.25 (d, *J* = 9.3 Hz, 1H), 8.15 (d, *J* = 9.3 Hz, 1H), 7.68 (d, *J* = 7.2 Hz, 2H), 7.60 (dd, *J* = 8.3, 4.4 Hz, 1H), 7.36 (d, *J* = 7.6 Hz, 2H), 2.87 (s, 3H), 2.47 (s, 3H).
   ¹³C NMR (75 MHz, CDCl₃) δ 153.31, 152.55, 151.32, 148.88, 139.88, 139.13, 138.21, 136.06, 132.55, 132.24, 129.84, 129.28 (2C), 129.14 (2C), 126.41, 121.98, 24.23, 21.35. MS (ESI) m/z: 286.2 ([M+H]+, 100). **Masse haute résolution (ESI) :** m/z calculé pour [M+H]⁺ C₁₉H₁₆N₃ : 286.1344; m/z mesuré : 286.1344. **Pureté (HPLC/UV λ at 260 nm):** 100%.
**Ibf : Rendement:** 51% (25.2 mg). **RMN ¹H (300 MHz, CDCl₃)** δ 9.41 (d, *J* = 8.3 Hz, 1H), 9.00 (brs, 1H), 8.20 (d, *J* = 9.2 Hz, 1H), 8.10 (d, *J* = 9.2 Hz, 1H), 7.52 (dd, *J* = 7.9, 3.8 Hz, 1H), 6.91 (s, 2H), 3.89 (s, 9H), 2.82 (s, 3H). **RMN ¹³C (75 MHz, CDCl₃)** δ 153.23, 153.11, 152.47, 151.42 (br), 148.90 (br), 140.06, 138.87, 138.02, 134.27, 132.54 (2C), 129.80, 126.29(br), 122.06, 106.67 (2C), 60.96, 56.31 (2C), 24.27. MS (ESI) m/z: 362.2 ([M+H]⁺, 100). **Masse haute résolution (ESI):** m/z calculé pour [M+H]⁺ C₂₁H₂₀N₃O₃ : 362.1505; m/z mesuré : 362.1503. **Pureté (HPLC/UV λ at 260 nm):** 100%.
**Ibo : Rendement:** 64% (30.4 mg). **RMN ¹H (300 MHz, CDCl₃)** δ 9.47 (d, *J* = 8.2 Hz, 1H), 9.09 (s, 1H), 8.29 (d, *J* = 8.8 Hz, 1H), 8.18 (d, *J* = 7.8 Hz, 1H), 7.75 (d, *J* = 8.5 Hz, 2H), 7.66 (brs, 1H), 7.55 (d, *J* = 8.5 Hz, 2H), 2.87 (s, 3H). **RMN ¹³C (100 MHz, CDCl₃)** δ 152.29, 152.05, 151.78 - 149.58 (br), 149.59 - 148.60 (br), 140.28, 138.33, 137.35, 135.39, 132.85, 132.48, 130.76 (2C), 128.75 (2C), 123.16 - 121.57 (br), 24.15. **MS (ESI) m/z:** 306.1, ([M+H]⁺, 100), 308.1 ([M+H]⁺, 40). **Masse haute résolution (ESI) :** m/z calculé pour [M+H]⁺ C₁₈H₁₃ClN₃ : 306.0798; m/z mesuré : 306.0800. **Pureté (HPLC/UV λ at 260 nm):** 100%.
**Ibp : Rendement:** 50% (196.0 mg). **RMN ¹H (400 MHz, CDCl₃)** δ 9.46 (dd, *J* = 8.2, 1.2 Hz, 1H), 9.08 (s, 1H), 8.30 (d, *J* = 9.3 Hz, 1H), 8.17 (d, *J* = 9.3 Hz, 1H), 7.91 (d, *J* = 1.2 Hz, 1H), 7.68 - 7.60 (m, 3H), 2.88 (s, 3H). **RMN ¹³C (101 MHz, CDCl₃)** δ 152.09, 151.69, 150.71, 149.01, 140.53, 138.80, 133.60, 133.16, 132.94, 132.53, 131.44, 130.45, 129.78, 128.56, 126.29, 122.30, 24.09. **MS (ESI) m/z :** 242.1 ([M+H]⁺, 100) et 344.1 ([M+H]⁺, 50). **Masse haute résolution (ESI) :** m/z calculé pour [M+H]⁺ C₁₈H₁₂Cl₂N₃ : 340.0408; m/z mesuré : 340.0405. **Purity (HPLC/UV λ at 264 nm):** 94%.
**Ibh : Rendement:** 52% (19.0 mg). **RMN ¹H (300 MHz, CDCl₃)** δ 9.46 (d, *J* = 8.3 Hz, 1H), 9.06 (s, 1H), 8.27 (d, *J* = 9.3 Hz, 1H), 8.16 (d, *J* = 9.3 Hz, 1H), 7.78 (ddd, *J* = 8.7, 5.3 Hz, 2H), 7.63 (dd, *J* = 8.1, 4.2 Hz, 1H), 7.26 (t, J = 8.6 Hz, 2H), 2.87 (s, 3H). **RMN ¹³C (75 MHz, CDCl₃)** δ 163.28 (d, *J* = 249.5 Hz), 152.32, 152.18, 151.49, 148.91, 140.10, 138.20, 134.93 (d, *J* = 3.3 Hz, 2C), 132.60, 132.50, 131.32 (d, *J* = 8.2 Hz, 2C), 129.80, 126.31, 122.11, 115.55 (d, *J* = 21.7 Hz), 24.21. **MS (ESI) m/z:** 290.2 ([M+H]⁺, 100). **Masse haute résolution (ESI):** m/z calculé pour [M+H]⁺ C₁₈H₁₃N₃F : 290.1094; m/z mesuré : 290.1094. **Pureté (HPLC/UV λ at 260 nm):** 100%.
**Ibq : Rendement:** 63% (34.5 mg). **RMN ¹H (400 MHz, CDCl₃)** δ 9.52 (dd, *J* = 8.2, 1.6 Hz, 1H), 9.07 (dd, *J* = 4.4, 1.6 Hz, 1H), 8.29 (d, *J* = 9.3 Hz, 1H), 8.19 (d, *J* = 9.3 Hz, 1H), 7.89 (d, *J* = 8.5 Hz), 7.80 (d, J = 8.5, 2H), 7.70 (dd, *J* = 8.5, 1.2 Hz, 2H), 7.64 (dd, *J* = 8.2, 4.4 Hz, 1H), 7.50 (t, *J* = 7.5 Hz, 2H), 7.41 (tt, *J* = 7.5, 1.2 Hz, 1H), 2.94(s, 3H). **RMN ¹³C (101 MHz, CDCl₃)** δ 152.93, 152.57, 151.46, 148.97, 142.01, 140.40, 140.11, 138.33, 137.79, 132.60, 132.53, 129.88 (3C), 128.92 (2C), 127.75, 127.20 (4C), 126.45, 122.09, 77.00, 24.30. **MS (ESI)** m/z: 348.2 ([M+H]⁺, 100). **Masse haute résolution (ESI):** m/z calculé pour [M+H]⁺ C₂₄H₁₇N₃ : 340.0408; m/z mesuré : 340.0405. **Pureté (HPLC/UV λ at 260 nm):** 100%.
**Ibj : Rendement:** 61% (30.1 mg). **RMN ¹H (400 MHz, CDCl₃)** δ 9.57 (d, *J* = 6.7 Hz, 1H), 9.42 - 8.68 (brs, 1H), 8.35 (brs, 1H), 8.25 (s, 1H), 8.24 (brs, 1H), 8.04 (d, *J* = 8.4 Hz, 1H), 7.98 (dd, *J* = 6.4 and 5.0 Hz, 1H), 7.96 (dd, *J* = 6.4 and 5.0 Hz, 1H),, 7.91 (dd, *J* = 8.5, 1.6 Hz, 1H), 7.74 (m, 1H), 7.63 - 7.55 (m, 2H), 2.95 (s, 3H). **RMN ¹³C (101 MHz, CDCl₃)** δ 153.35, 152.81, 140.17, 136.34, 133.40, 133.04, 129.82, 129.08, 128.50, 128.24, 127.81, 127.02, 126.72, 126.66, 24.32. **MS (ESI) m/z:** 322.2 ([M+H]⁺, 100). **Masse haute résolution (ESI) :** m/z calculé pour [M+H]⁺ C₂₄H₁₈N₃ : 348.1501; m/z mesuré : 348.1499. **Pureté (HPLC/UV λ at 260 nm):** 100%.

### Exemple 2 : Synthèse du composé lad

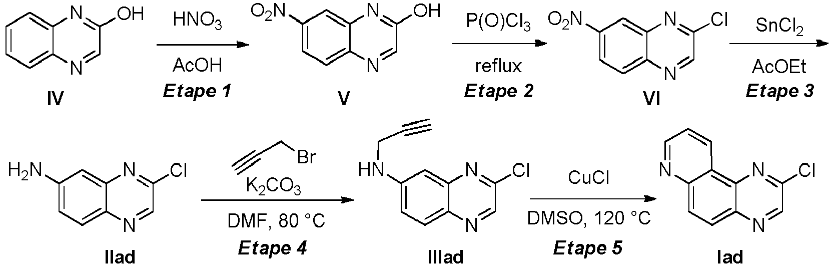

Etape 1 : A une solution de 2-quinoxalinol (20,0 g, 0,137 mol, 1 equiv.) commerciale dans l'acide acétique (200 mL) placé à 0°C, est ajoutée goutte à goutte une solution d'acide nitrique à 70 % (17,4 mL, 0,274 mol, 2 equiv.) diluée dans 20 mL d'acide acétique. Le milieu réactionnel est ensuite laissé remonter à température ambiante. Après 3 h à température ambiante, la couleur du milieu réactionnel passe de marron à jaune-orangé. précipité est récupéré par filtration et lavé à l'eau. Le solide de couleur jaune-orangé est séché sous vide pendant 48 h pour donner 17,57 g de composé **V** (67%). **RMN ¹H MHz, Acetone-d₆)** δ ppm 9.14 (s, 2H), 8.93 (d, *J* = 2.5 Hz, 1H), 8.61 (dd, *J* = 9.1, 2.5 Hz, 1H), 8.35 (d, *J* = 9.1 Hz, 1H).
Etape 2 : A une solution du composé **V** (17,570 g, 91,50 mmol, 1 equiv.) dans 80 mL de POCl₃ sont ajoutées 30 gouttes de diméthylformamide. Le milieu réactionnel est ensuite placé pendant 3 h à reflux. La couleur du milieu réactionnel devient noire. Après refroidissement le milieu réactionnel est versé lentement dans un bécher de 500 mL rempli de glace pilée. Le précipité est récupéré par filtration et lavé à l'eau. Le solide obtenu est séché sous vide pendant 48 h pour donner le composé **VI** sous forme d'un solide gris (17,02 g, 88%). **RMN ¹H (300 MHz, CDCl₃) δ ppm** : 8.30 (d, *J* = 9.3 Hz, 1H), 8.56 (dd, *J* = 9.3 et 2.2 Hz, 1H), 8.92 (d, *J* = 2.5 Hz, 1H), 8.93 (s, 1H). **RMN ¹³C (75 MHz, CDCl₃) δ ppm :** 123.7, 124.8, 131.1, 141.1, 143.2, 148.0, 148.7, 149.8.
Etape 3 : A une suspension de composé nitré **VI** (14,36 g, 68,5 mmol, 1 equiv.) dans l'AcOEt (300 mL) est ajouté SnCl₂.2H₂O (45,5g, 239,9 mmol, 3,5 equiv.), puis le mélange réactionnel est mis à reflux pendant 2h. Après refroidissement, NaOH 50% (6 equiv., 480 mmol) est aditionné lentement à 0°C et le milieu réactionnel est filtré sur un pad de gel de silice, puis on élue avec de l'acétone à chaud. Après concentration, le résidu est purifié par recristallisation avec CHCl₃ / Ether de petrole pour fournir le composé **IIad** sous forme d'un solide jaune (9,65g, 78%). **RMN ¹H (300 MHz, CDCl₃) δ ppm** : 4.30 (brs, 2H), 7.03 (d, J = 1.7 Hz, 1H), 7.15 (dd, J = 8.8, 1.7 Hz, 1H), 7.85 (d, J = 8.8 Hz, 1H), 8.47 (s, 1H). **RMN ¹³C (75 MHz, CDCl₃) δ ppm :** 107.2, 121.7, 130.3, 135.98, 140.3, 144.2, 147.7, 149.1. **Masse haute résolution (ESI) :** m/z calculé pour [M+H]⁺ C₈H₇N₃Cl : 180.0329 ; m/z mesuré : 180.0326.
Etape 4 : Le composé **IIIad** a été préparé à partir du composé **IIad** selon la procédure décrite à l'exemple 1.
Etape 5 : Le composé **lad** a été préparé à partir du composé **IIIad** selon la procédure décrite à l'exemple 1. **RMN ¹H (300 MHz, CDCl₃)** δ 9.40 (ddd, *J* = 8.3, 1.7, 0.6 Hz, 1H), 9.12 (dd, *J* = 4.4, 1.7 Hz, 1H), 8.91 (s, 1H), 8.31 (dd, *J* = 9.3, 0.6 Hz, 1H), 8.20 (d, *J* = 9.3 Hz, 1H), 7.69 (dd, *J* = 8.3, 4.4 Hz, 1H). **RMN ¹³C (75 MHz, CDCl₃)** δ 152.52, 149.36, 147.42, 144.95, 140.28, 140.13, 133.10, 132.92, 129.73, 125.43, 122.53. **IR :** 3049, 1493, 1207,1146, 900, 841.

### Exemple 3 : Synthèse du composé Iaj

Une suspension du composé chloré **lad** (100 mg, 0,464 mmol, lequiv.), de K₂CO₃ (16,8 mg, 1,186 mmol, 2,6 equiv.), d'acide 2-naphtyl-boronique (102 mg, 0,593mmol, 1,3 equiv.) et de PdCl₂(PPh₃)₂ (10 mg, 0,0139 mmol, 0,03 equiv.) dans un mélange dioxane / H₂O (4 mL/1 mL) est chauffé à reflux pendant 1h. Après refroidissement le milieu réactionnel est extrait trois fois avec de l'acétate d'éthyle, lavé une fois avec une solution de 10% de K₂CO₃. Après séchage sur MgSO₄, filtration et concentration, le résidu est purifié sur colonne de silice (eluant CH₂Cl₂ : AcOEt, 77 : 33) pour donner le composé **Iaj** sous forme d'un solide jaune pale (121 mg, 85%). **RMN ¹H (300 MHz, CDCl₃) δ ppm :** 7.58 (dd, J = 3.5, 6.6 Hz, 1H), 7.60 (dd, J = 3.5, 6.6 Hz, 1H), 7.73 (dd, J = 8.2, 4.3 Hz, 1H), 7.94 (m, 1H), 8.05 (t, J = 4.3 Hz, 1H), 8.07 (d, J = 8.2 Hz, 1H), 8.26 (d, J = 9.4 Hz, 1H), 8.30 (d, J = 9.4 Hz, 1H), 8.50 (dd, J = 9.6, 1.6 Hz, 1H), 8.76 (brs, 1H), 9.13 (dd, J = 4.5, 1.6 Hz, 1H), 9.60 (s, 1H), 9.70 (dd, J = 8.2, 1.7 Hz, 1H). **RMN ¹³C (75 MHz, CDCl₃) δ ppm:** 122.2, 124.4, 126.6, 126.8, 127.4, 127.8, 128.9, 129.1, 130.3, 132.9, 133.4, 133.9, 134.2, 140.0, 140.7, 143.1, 149.5, 150.7, 151.97, 152.0. **IR :** 1728, 1701, 1539, 1499, 1380 1287, 1237, 1210, 1090, 1016, 845, 825, 791, 751, 732. **Masse haute résolution (ESI)** : m/z calculé pour [M+H]⁺C₂₁H₁₄N₃ : 308.1188 ; m/z mesuré : 308.1188.

Les composés suivant ont également été synthétisés selon cette procédure :
**Iac : RMN ¹H (400 MHz, CDCl₃)** *δ* 9.66 (d, *J* = 8.3 Hz, 1H), 9.46 (s, 1H), 9.15 (brs, 1H), 8.33 (dd, *J* = 8.3 et 1.5 Hz, 2H), 8.26 (t, *J* = 8.5 Hz, 2H), 7.72 (brm, 1H), 7.61 (m, 3H). **RMN ¹³C (100 MHz, CDCl₃)** *δ* 122.3 (br), 126.8 (br), 127.5 (2C), 129.2 (2C), 130.2, 130.3, 132.4, 132.8, 136.5, 140.1, 140.7, 142.9, 149.7 (br), 150.7, 151.9 (br). **Masse haute résolution (ESI) :** m/z calculé pour [M+H]⁺ C₁₇H₁₂N₃ : 258.1031 ; m/z mesuré : 258.1028.
**Iae : RMN ¹H (300 MHz, DMSO)** δ 9.80 (brs, 1H, OH), 9.65 (s, 1H), 9.56 (dd, *J* = 8.3, 1.7 Hz, 1H), 9.14 (dd, *J* = 4.3, 1.7 Hz, 1H), 8.26 (s, 2H), 7.93-7.87 (m, 2H), 7.88 (dd, *J* = 8.4, 4.4 Hz, 1H), 7.44 (t, *J* = 8.2 Hz, 1H), 7.01 (ddd, *J* = 8.2, 2, 1 Hz 1H).
   **RMN ¹³C (75 MHz, DMSO)** δ 158.14, 152.20, 150.10, 148.75, 143.50, 140.15, 138.90, 137.17, 132.07, 131.99, 130.26, 130.03, 125.76, 122.78, 118.26, 117.67, 114.04, 39.52. **Masse haute résolution (ESI)** : m/z calculé pour [M+H]⁺ C₁₇H₁₂N₃O : 274.0980 ; m/z mesuré : 274.0978.
**Iaf : RMN ¹H (300 MHz, CDCl₃)** δ 9.59 (ddd, *J* = 8.3, 1.7, 0.5 Hz, 1H), 9.39 (s, 1H), 9.12 (dd, *J* = 4.4, 1.7 Hz, 1H), 8.29 (dd, *J* = 9.4, 0.5 Hz, 1H), 8.23 (d, *J* = 9.4 Hz, 1H), 7.71 (dd, *J* = 8.3, 4.4 Hz, 1H), 7.55 (s, 2H), 4.06 (s, 6H), 3.97 (s, 2H). **RMN ¹³C (75 MHz, CDCl₃)** δ 154.11, 152.12, 150.63, 149.67, 142.97, 140.79, 140.66, 139.96, 132.91, 132.47, 132.16, 130.44, 126.63, 122.31, 105.11, 61.22, 56.62. **IR** 3000-2837, 1588, 1494, 1343, 1235, 1127, 853. **Masse haute résolution (ESI) :** m/z calculé pour [M+H]⁺ C₂₀H₁₈N₃ : 348.1348; m/z mesuré : 348.1353.
**Iah : RMN ¹H (300 MHz, CDCl₃)** δ 9.58 (ddd, *J* = 8.3, 1.7, 0.6 Hz, 1H), 9.38 (s, 1H), 9.10 (dd, *J* = 4.4, 1.7 Hz, 1H), 8.31 (dd, *J* = 9.0, 5.4 Hz, 2H), 8.27 (dd, *J* = 9.3, 0.6 Hz, 2H), 8.20 (d, *J* = 9.3 Hz, 2H), 7.68 (dd, *J* = 8.3, 4.4 Hz, 1H), 7.28 (t, *J* = 8.5 Hz, 2H). **RMN ¹³C (75 MHz, CDCl₃)** δ 164.35 (d, *J*= 251.1 Hz), 152.00, 149.58 (d, *J* = 14.0 Hz), 142.49, 140.64, 139.84, 132.74, 132.48, 130.19, 129.40 (d, *J* = 8.5 Hz), 126.47, 122.18, 116.30 (d, *J* = 21.7 Hz). **RMN ¹⁹F (188 MHz, CDCl₃)** δ 110.66 (tt, J = 8.5, 5.4 Hz). **Masse haute résolution (ESI) :** m/z calculé pour [M+H]⁺ C₁₇H₁₁N₃F : 276.0937 ; m/z mesuré : 276.0937.
**Iam : RMN ¹H (300 MHz, DMSO)** δ 10.01 (d, *J* = 1.9 Hz, 1H), 9.95 (s, 1H), 9.71 (dd, *J* = 8.3, 1.5 Hz, 1H), 9.49 (d, *J* = 2.0 Hz, 1H), 9.17 (dd, *J* = 4.2, 1.5 Hz, 1H), 8.31 (s, 2H), 8.21 (d, *J* = 7.4 Hz, 1H), 8.15 (d, *J* = 8.3 Hz, 1H), 7.94 - 7.85 (m, 2H), 7.74 (ddd, *J* = 7.9, 6.9, 0.9 Hz, 1H). **RMN ¹³C (75 MHz, DMSO)** δ 152.40, 149.21, 148.80, 148.27, 148.09, 143.86, 140.47, 139.10, 135.05, 132.57(2C), 130.97, 130.05, 129.08, 128.88, 128.67, 127.49, 127.33, 125.76, 122.94, 39.52. **IR :** 3048,1572, 1498, 1300, 1090, 1066, 904, 847, 790, 753. **Masse haute résolution (ESI) :** m/z calculé pour [M+H]⁺ C₂₀H₁₃N₄ : 309.1140 ; m/z mesuré : 309.1140.
**Iai : RMN ¹H (300 MHz, CDCl₃)** δ 9.57 (ddd, *J* = 8.3, 1.7, 0.6 Hz, 1H), 9.29 (s, 1H), 9.12 (dd, *J* = 4.4, 1.8 Hz, 1H), 8.37 (dd, *J* = 9.3, 0.5 Hz, 1H), 8.34 (m, 1H), 8.31(dd, *J =* 9.3Hz, 1H), 8.05 (brd, *J* = 8.2 Hz, 1H), 8.00 (dd, *J* = 6.9, 2.5 Hz, 1H), 7.86 (dd, *J* = 7.1, 1.2 Hz, 1H), 7.72 - 7.63 (m, 2H), 7.63 - 7.51 (m, 2H). **RMN ¹³C (75 MHz, CDCl₃)** δ 153.32, 152.00, 149.43, 146.47, 140.46, 139.92, 134.98, 134.12, 132.97, 132.79, 131.21, 130.32, 130.27, 128.86, 128.69, 127.17, 126.61, 126.36, 125.41, 125.13, 122.32. IR 3000-2800, 1755, 1586, 1574, 1495, 1377, 1317, 1303, 1088, 1055, 899, 844, 795, 775, 732. **Masse haute résolution (ESI) :** m/z calculé pour [M+H]⁺ C₂₁H₁₄N₃ : 308.1188; m/z mesuré : 308.1187.
**Iag : RMN ¹H (300 MHz, CDCl₃)** δ 9.61 (ddd, *J* = 8.3, 1.7, 0.6 Hz, 1H), 9.55 (d, *J* = 1.7 Hz, 1H), 9.46 (s, 1H), 9.13 (dd, *J* = 4.4, 1.8 Hz, 1H), 8.80 (dd, *J* = 4.7, 1.2 Hz, 1H), 8.61 (ddd, *J* = 8.0, 2.2, 1.7 Hz, 1H), 8.32 (dd, *J* = 9.3, 0.5 Hz, 1H), 8.25 (d, *J* = 9.3 Hz, 1H), 7.72 (dd, *J* = 8.3, 4.4 Hz, 1H), 7.54 (ddd, *J* = 8.0, 4.9, 0.7 Hz, 1H). **RMN ¹³C (75 MHz, CDCl₃)** δ 152.21, 151.12, 149.52, 148.76, 148.38, 142.45, 141.28, 140.12, 134.67, 133.17, 132.78, 132.26, 130.18, 126.43, 123.90, 122.40. **IR :** 3040, 1614, 1589, 1496, 1477, 1391, 1304, 1289, 1095, 1075, 845, 790, 702. **Masse haute résolution (ESI) :** m/z calculé pour [M+H]⁺C₁₆H₁₁N₄ : 259.0984; m/z mesuré : 259.0984.

### Exemple 4 : Synthèse du composé Iak

A une solution du composé chloré **lad** (100 mg, 0,464 mmol, 1equiv.) dans le THF 5 mL, est additionné du trimethylsilylacétylène (260µL, 1,85 mmol, 4 equiv.), du CuI (4,3mg, 0,0232 mmol, 0,05 equiv.), du PdCl₂(PPh₃)₂ (6,5 mg, 0,0093mmol, 0,02 equiv) puis de la triéthylamine (3 mL). Le mélange réactionnel est porté à reflux sous atmosphère d'argon pendant 48 h, puis refroidit à température ambiante. Le mélange réactionnel est filtré sur pad de silice, et élué à l'acétate d'éthyle. Après concentration, le résidu est repris dans le méthanol (10 mL), du carbonate de potassium est ajouté (500 mg) et le mélange est porté 10 min à reflux. Après refroidissement le mélange réactionnel est dilué avec de l'eau (20 mL), extrait à l'AcOEt trois fois (30 mL) et lavé avec de la saumure deux fois (30 ml). Après séchage sur MgSO₄ et filtration, le mélange réactionnel est purifié sur gel de silice pour donner un solide brun correspondant au composé **Iak** (48 mg, 50%). **RMN ¹H (300 MHz, CDCl₃) δ ppm :** 3.50 (s, 1H), 7.70 (dd, J = 8.3, 4.3 Hz, 1H), 8.20 (d, J = 9.4 Hz, 1H), 8.32 (d, J = 9.4 Hz, 1H), 9.04 (s, 1H), 9.11 (dd, J = 4.4, 1.7 Hz, 1H), 9.50 (dd, J = = 8.2, 1.7 Hz, 1H). **RMN ¹³C (75 MHz, CDCl₃) δ ppm :** 81.0, 81.6, 122.6, 125.9, 130.1, 133.0, 133.9, 137.4, 140.2, 140.8, 147.7, 149.3, 152.3.

### Exemple 5 : Synthèse du composé Xbc

Etape 1 : A une solution de composé **IIbc** (500.0 mg, 2.13 mmol, 1 equiv.) dans un mélange THF/eau (10.5 / 1 mL) en présence de 10 mg de CuCl₂ (quantité catalytique) et 10 mg de cuivre (quantité catalytique) sous atmosphère inerte d'azote sont ajoutés 0,41 mL de triéthylamine (2,98 mmol, 1,4 equiv.) et 0,34 mL de 3-chloro-3-méthyl-1-butyne (2.98 mmol, 1.4 equiv.). Le milieu réactionnel est agité à température ambiante pendant 12 h, hydrolysé par 15 mL d'une solution saturée de K₂CO₃, puis extrait 3 fois au DCM. Les phases organiques sont rassemblées, lavées par une solution saturée de NaCl, séchées sur Na₂SO₄ puis concentrées sous préssion réduite. Une purification par chromatographie sur gel de silice dans un mélange DCM : acétate d'éthyle en proportions 85 : 15 a permis d'obtenir le composé *N*-alkylé correspondant (**9bc**). **Rendement** : 65 % (414.0 mg). **RMN ¹H (400 MHz, CDCl₃) δ ppm** : 1.68 (s, 6H), 2.40 (s, 1H), 2.66 (s, 3H), 4.32 (brs, 1H, NH), 7.16 (dd, *J* = 9.0, 2.5 Hz, 1H), 7.41-7.50 (m, 3H), 7.56 (d, *J* = 2.5 Hz, 1H), 7.61 (d, *J* = 8.3 Hz, 2H), 7.81 (d, *J* = 9.0 Hz, 1H). **RMN ¹³C (100 MHz, CDCl₃) δ ppm :** 23.6, 30.0 (2C), 47.8, 71.2, 86.4, 108.0, 123.2, 128.4 (2C), 128.5, 128.6, 128.8 (2C), 136.1, 139.6, 142.6, 145.8, 147.8, 154.6. **MS (ESI) m/z :** 302,3 ([M+H]⁺, 100). **Masse haute résolution (ESI) :** m/z calculé pour [M+H]⁺ C₂₀H₂₀N₃ : 302.1657 ; m/z mesuré : 302.1656. **Pureté (HPLC/UV λ à 254 nm)** : 95 %.
Etape 2 : Une solution de composé **9bc** (300,0 mg, 1,00 mmol, 1 equiv.) en présence de mg de CuCl dans le toluène (5 mL) est mise au reflux sous atmosphère inerte d'azote pendant 1 h. Après refroidissement, le milieu réactionnel est hydrolysé par 10 mL d'une solution saturée de K₂CO₃ puis extrait au DCM. Les phases organiques rassemblées, sont lavées par une solution saturée de NaCl, séchées sur Na₂SO₄, puis évaporées sous pression réduite. Une purification par chromatographie sur gel de silice dans un mélange DCM : AcOEt en proportions 10 : 0 jusque 8 : 2 a permis d'obtenir le composé **Xbc. Rendement** 64 % (191.0 mg). **RMN ¹H (400 MHz, CDCl₃) δ ppm :** 1.38 (s, 6H), 2.69 (s, 3H), 4.07 (brs, NH, 1H), 5.52 (d, *J* = 9.8 Hz, 1H), 6.93 (d, *J* = 8.8 Hz, 1H), 7.42 (d, *J* = 9.8 Hz, 1H), 7.45-7.52 (m, 3H), 7.65 (d, *J* = 8.8 Hz, 1H), 7.70 (dd, *J* = 8.0, 1.5 Hz, 2H). **RMN ¹³C (100 MHz, CDCl₃) δ ppm** : 23.8, 31.4 (2C), 52.6, 111.7, 118.8, 119.9, 128.0, 128.2 (2C), 128.6, 128.7, 129.2 (2C), 135.7, 138.3, 139.9, 143.0, 147.0, 153.7. **MS (ESI) m/z :** 302.3 ([M+H]⁺, 100).

### II. Evaluation biologique

Les composés selon l'invention ont été évalués pour leurs propriétés neuroprotectrices et neurodifférentiatrices (ou neuritogéniques).

### II.1 Effet des composés sur la neuroprotection et la différenciation des neurones dopaminergiques issus de cultures primaires de mésencéphale ventral embryonnaire.

Les composés selon l'invention ont été testés dans un modèle de dégénérescence des neurones dopaminergiques issus de cultures primaires de mésencéphale ventral embryonnaire selon le protocole décrit dans l'article de S. Guerreiro *et al.* (2008). Les cultures primaires de mésencéphale ventral embryonnaire ainsi obtenues sont des cultures mixtes contenant environ 50 % de cellules neuronales et 50 % de cellules gliales. Parmi cellules neuronales, les neurones dopaminergiques représentent 3% des neurones totaux, majorité des neurones issus de ces cultures étant GABAergiques. Concernant les cellules gliales, elles sont principalement représentées par des astrocytes (> de 95 % des cellules gliales). Ces cultures sont caractérisées par la mort spontanée, progressive et sélective des neurones dopaminergiques en conséquence d'un mécanisme impliquant des astrocytes immatures et une dyshoméostasie calcique (E. Rousseau *et al.* (2013), D. Toulorge *et al.* (2011)). L'effet neuroprotecteur des composés selon l'invention a été évalué par comptage des neurones dopaminergiques (TH+) marqués par immunohistochimie de la tyrosine hydroxylase (TH) après 10 jours de culture (DIV 10). L'observation à DIV 10 est directement liée au fait que, d'après la littérature, une grande diminution du nombre de neurons TH+ est observé après 10 jours de cultures en l'absence de traitement neuroprotecteur.

Les résultats obtenus sont présentés dans le tableau 1 ci-dessous.

**Tableau 1. Activité neuroprotectrice de composés selon l'invention sur la survie des neurones dopaminergiques issus de mésencéphale ventral embryonnaire.**

| Composés | % relatif par rapport au contrôle ± erreur standard | | |
|---|---|---|---|
| | Neurones TH^{+a} 100 nM | Neurones TH^{+a} 1 µM | Neurones TH^{+a} 10 µM |
| Contrôle | | 26,0 ± 0,5 | |
| db-AMPc^{b} | | 100,0 ± 1,4 | |
| **Iac** | 46,6 ± 2,8 | 64,7 ± 2,9* | 91,0 ± 3,7* |
| **Iaa** | 25,7 ± 1,6 | 26,2 ± 1,7 | 30,6 ± 2,3 |
| **Iad** | 27,4 ± 1,6 | 26,5 ± 1,6 | 33,8 ± 1,8 |
| **Iae** | 24,7 ± 1,6 | 47,7 ± 2,6* | 99,3 ± 4,4* |
| **Iaf** | 27,7 ± 1,6 | 46,8 ± 2,3* | 70,8 ± 3,5* |
| **Iag** | 31,4 ± 1,7 | 48,3 ± 2,4* | 96,6 ± 4,5* |
| **Iam** | 27,6 ± 2,0 | 46,7 ± 2,7* | 97,3 ± 3,6* |
| **Iah** | 24,4 ± 1,4 | 32,8 ± 1,8 | 42,8 ± 2,2* |
| **Iaj** | 28,9 ± 1,8 | 42,8 ± 2,1* | 94,9 ± 3,5* |
| **Iak** | 38,2 ± 2,1* | 40,7 ± 2,6* | 7,0 ± 1,3* |
| **Ibj** | 58,1 ± 2,8 | 59,2 ± 2,2 | 67,3 ± 2,5* |

| | | | |
|---|---|---|---|
| ^{a}Nombre de neurones TH⁺ par puits exprimé en pourcentage par rapport aux cultures traitées avec le db-AMPc à 1 mM. L'écart standard à la moyenne est obtenu sur trois expériences indépendantes pour les composés actifs dont les conditions sont répliquées trois fois. ^{b}db-AMPc utilisé à une concentration de 1 mM. Analyses statistiques : *P<0,05, *vs* contrôle, analyse de variance à un facteur (one-way ANOVA) suivi d'une analyse *post hoc* de Dunnett. | | | |

Les résultats montrent une activité particulièrement importante des composés **lac, Iae**, **Iag, Iaj** et **Iam** avec un pourcentage de survie quasiment égal à 10µM à celui induit par la db-AMPc à 1 mM.

### II.2 Etude du passage de la barrière hémato-encéphalique du composé Iac

Le composé **Iac** est une 3-phényl-1,4,8-triazaphénanthrène. *In vitro,* il possède une activité neuroprotectrice à une concentration de 1µM pour une activité optimal à 10µM.

Les propriétés physicochimiques du composé **Iac** ont été comparées aux propriétés physicochimiques théoriques attendues pour un composé actif au niveau central puis le passage de la barrière hémato-encéphalique a été évalué.

### II.2.1 Prédiction de la capacité d'une molécule à traverser passivement la BHE du SNC (QSARs)^{a}

Une comparaison des propriétés physico-chimiques de **Iac** avec celles des composés actifs sur le SNC est présenté dansle tableau 2 ci-dessous.

**Tableau 2. Comparaison des propriétés physico-chimiques de Iac avec celles des composés actifs sur le SNC.**

| Propriétés physicochimiques | Médicaments du SNC^{a} | Composé **Iac** |
|---|---|---|
| Activité | < nM | 1 µM |
| Sélectivité | Élevée | Inconnue |
| Poids moléculaire | < 450 g.mol⁻¹ | 257 g.mol⁻¹ |
| logP | < 5 | 2,4^{b} |
| Donneur de liaisons H | < 3 | 0 |
| Accepteur de liaisons H | < 7 | 3 |
| Liaisons rotatives | < 8 | 1 |
| pKa | 7,5-10,5 (éviter les acides) | indéterminé |
| Aire polaire de surface (PSA) | < 60-70 A° | 39 A°^{c} |
| Solubilité aqueuse | > 60 µg.mL⁻¹ | < 10 µg.mL⁻¹ |

| | | |
|---|---|---|
| ^{a}Voir Pajouhesh et Lenz (2005). ^{b}Les prédictions des logP ont été réalisées avec l'application en ligne gratuite ALOGP Computational Chemistry Laboratory, http://www.vcclab.org, 2005. ^{c}Valeur calculée à l'aide de molinspiration (www.molinspiration.com) | | |

Le composé **Iac** possède, en termes de propriétés physicochimiques, la plupart des caractéristiques nécessaires à sa diffusion à travers la BHE. Cependant ce type de prédiction est limité à la diffusion passive de la molécule et ne prend pas en compte d'éventuelles interactions avec la barrière, telles que la métabolisation par les cytochromes de la BHE, ainsi que les phénomènes de pompes à efflux ou de transport actif. Une étude *in vivo* de passage de la BHE est donc indispensable pour confirmer cette prédiction.

### II.2.2 Étude in vivo du passage de la BHE du composé Iac

Le but de cette étude est de vérifier par HPLC couplée à une spectrométrie de masse, la présence du composé **Iac** dans le parenchyme cérébral après un traitement sub-chronique par voie intraveineuse chez la souris.

### Animaux :

L'étude est réalisée avec des souris CD-1.

Une solution du composé **Iac** est préparée à 0,5 mg/mL dans du sérum physiologique contenant 10% de Cremophor EL®. Une même dose de cette solution est administrée à 6 souris par voie intraveineuse (2 µL/g).

Après 5 min, 3 souris sont sacrifiées. Après 30 min, les 3 autres souris sont sacrifiées.

Le sang est prélevé et le plasma est séparé par centrifugation. Le cerveau est prélevé après perfusion intracardiaque. Les échantillons sont congelés et conservés à -80°C avant traitement.

### Traitement des échantillons :

Plasma : 400 µL de chaque échantillon de plasma sont mélangés avec 1 mL d'acétonitrile pour précipiter les protéines et extraire le composé **Iac.**

Cerveau : Chaque cerveau est broyé dans 400 µL de sérum physiologique. 800 µL d'acétonitrile sont ajoutés pour extraire le composé **Iac.**

### Extraction :

Les échantillons traités sont agités à l'aide d'un vortex pendant 3 minutes, puis placés dans un sonicateur pendant 3 minutes également. Les protéines précipitées et les résidus solides sont sédimentées par centrifugation (15 000g, 5 minutes à 4°C). Les surnageants sont transférés dans une microplaque pour être analysés par HPLC-MS/MS.

### Analyse par HPLC-MS/MS :

Les échantillons sont analysés avec la méthode décrite ci-dessous, la détection et la quantification, basées sur l'aire des pics, étant réalisées par HPLC-MS/MS en mode MRM. Les solutions des gammes étalons sont analysées dans la même série d'injections.

Pour le calcul du log [cerveau]/[plasma] permettant de déterminer le passage de la barrière hématoencéphalique, le volume de plasma moyen d'une souris est évalué à 1,5 mL.

Au préalable, une analyse FIA est réalisée comme décrit ci-dessous afin de choisir les conditions d'analyses pour la MS/MS. Elle est réalisée en injectant directement dans le spectromètre de masse le produit **Iac** pur dilué dans un solvant. Il n'y a donc pas de séparation en HPLC puisqu'il s'agit d'une solution témoin et non d'un extrait de cerveau. Une solution du composé **Iac** est injectée en flux continu dans le spectromètre masse, et tensions sont ajustées pour obtenir une intensité optimale pour l'ion moléculaire et un ou deux ions fils obtenus par fragmentation de l'ion moléculaire.

### Analyse par injection en flux continu (Flow injection Analysis - FIA) :

Une solution du composé **Iac** est préparée à 1 mg/mL dans l'acétonitrile. Cette solution est diluée au 1/100.

La mise au point de l'analyse est réalisée avec une UHPLC couplée à un triple quadripôle Shimadzu LC-MS 8030. Le système est utilisé en mode FIA. 1 µL de la solution diluée est injectée.

Les conditions d'analyse de l'ion moléculaire sont optimisées (source, ionisation). Le rapport m/z de l'ion moléculaire est ajusté. Les tensions sont ajustées (Q1, chambre de collision, Q3). Le ou les meilleurs fragments sont sélectionnés et leurs rapports m/z ajusté.

### Méthode HPLC-MS/MS en mode MRM :

Une solution du composé **Iac** à 10 mM dans le DMSO est diluée à une concentration de l'ordre du µM pour mettre au point la méthode d'analyse.

Les conditions chromatographiques sont optimisées (solvants, pH, mode d'élution, débit, etc.). Les chromatogrammes sont enregistrés en injectant de préférence 1 µL de solution.

### Étalonnage pour dosage dans le plasma :

Une gamme étalon est réalisée à partir de 5 dilutions du composé **Iac** dans du plasma de souris. Les échantillons dans le plasma sont traités et extraits comme indiqué précédemment.

### Recouvrement (plasma) :

La concentration considérée est de 1 µM (n = 2).

Les échantillons sont préparés de manière identique à celle pour l'étalonnage mais dans ce cas, le plasma ne contient pas de composé **Iac.** Ce dernier est présent dans l'acétonitrile en quantité suffisante pour arriver à une concentration équivalente à 1 µM dans du plasma. Étalonnage pour dosage dans le cerveau :
Une gamme étalon est réalisée à partir de 5 dilutions du composé **Iac** dans des cerveaux de souris broyés dans 400 µL de solution saline. Les échantillons dans des cerveaux sont traités et extraits comme indiqué précédemment.

### Recouvrement (cerveau) :

La quantité considérée est de 1 nmol (n = 2).

Les échantillons sont préparés de manière identique à celle pour l'étalonnage mais dans ce cas, le cerveau ne contient pas de composé **Iac.** Ce dernier est présent dans l'acétonitrile en quantité suffisante pour arriver à une quantité équivalente à 1 nmol par cerveau :
Une fois les étapes d'étalonnage et de recouvrement effectuées, les échantillons prélevés sur les animaux traités, puis traités et extraits comme indiqué précédemment, sont analysés.

### Résultat :

Les résultats obtenus sont présentés dans le tableau ci-dessous.

| Délais pour l'euthanasie après injection IV de 1 mg/kg de **Iac** | Concentration plasmatique (nmol/mL) | Quantité (nmol/cerveau) | Log [cerveau]/[plasma] |
|---|---|---|---|
| 5 min | 2,47 ± 0,24 | 5,19 ± 0,25 | 0,15 ± 0,05 |
| 30 min | 0,32 ± 0,05 | 0,50 ± 0,07 | 0,02 ± 0,10 |

### Conclusion :

Le composé I**ac** est capable de traverser *in vivo* la BHE après administration intraveineuse. Ces résultats confirment les prédictions QSARs.

### II.3 Essais in vivo.

Les effets neuroprotecteurs potentiels de Iac ont été évalués en mesurant les niveaux de dopamine du striatum et le renouvellement de la dopamine dans le modèle de souris de la maladie de Parkinson traitées à la MPTP (1-méthyl-4-phényl-1,2,3,6-tétrahydropyridine), ainsi qu'en évaluant la quantité de neurones dopaminergiques sauvés dans la substancia nigra. Les souris ont été traitées avec Iac (50 et 100 mg/kg, p.o., dans 1% carboxymethylcellulose et 0.5% de tween 80) pendant 11 jours consécutifs. MPTP (20 mg/kg; voie intrapéritonéale) ou une solution saline pour les controles a été administré aux jours de traitement 4 à 8. Toutes les souris ont été tuées le jour 12 suivant l'administration finale du traitement, puis le tissu du striatum a été disséqué pour analyse. L'effet du traitement à la MPTP sur la déplétion de la dopamine du striatum a été analysé par chromatographie en phase liquide haute pression (HPLC) en combinaison avec une électrochimique pour la mesure des niveaux de dopamine monoamine (DA), d'acide 3,4-dihydroxyphénylacétique (DOPAC) et d'acide homovanillique (HVA).

Iac (50 mg/kg et 100 mg/kg) a augmenté les niveaux de DA du striatum (64% et 78%, respectivement). Les niveaux de HVA du striatum ont également été augmenté (25%) mais seulement à la dose la plus élevée de Iac (100 mg/kg). Une diminution du rapport (DOPAC + HVA) / DA) a été observée aux doses de 50 mg/kg (30%) et 100 mg/kg (28%) de Iac. Il n'y a pas eu d'effet significatif du traitement par Iac sur les niveaux de DOPAC. A la dose de 2x25 mg/kg/j (*per os*), il a été observé la survie presque totale des neurones TH⁺. En effet, dans le groupe des souris traitées par MPTP/Iac, la survie a été mesurée à 93 ± 4% par rapport aux contrôles. A une dose plus élevée (2x50 mg/kg/j), Iac est un peu moins actif puisque la survie des neurones dopaminergiques est de 81 ± 4% par rapport aux contrôles.

Ces données suggèrent que le traitement à Iac permet de neutraliser une partie de la perte de dopamine et d'atténuer l'augmentation du renouvellement de la dopamine, et de sauvegarder les neurones dopaminergiques de la substance noire.

### REFERENCES BIBLIOGRAPHIQUES

S. Guerreiro, et al. (2008) : « Paraxanthine, the primary metabolite of caffeine, provides protection against dopaminergic cell death via stimulation of ryanodine receptor channels » Mol. Pharmacol. 2008, 74 (4), 980e989.
Mourlevat et al. (2003) : « Prévention of Dopaminergic Neuronal Death by Cyclic AMP in Mixed Neuronal/Glial Mesencephalic Cultures Requires the Repression of Presumptive Astrocytes » Molecular Pharmacology 2003, 64:578-586.
Pajouhesh et Lenz (2005) : « Médicinal Chemical Properties of Successful Central Nervous System Drugs » NeuroRx. 2005, 2: 541-553.
E. Rousseau, et al. (2013) : « The iron-binding protein lactoferrin protects vulnérable dopamine neurons from degeneration by preserving mitochondrial calcium homeostasis » Mol. Pharmacol. 2013, 84, 888e898. D. Toulorge, et al. (2011) : « Neuroprotection of midbrain dopamine neurons by nicotine is gated by cytoplasmic Ca2+ » FASEB J. 2011, 25, 2563e2573.
WO 2010/007179
WO 2012/131080

## Revendications

1. Composé de formule (I) suivante : 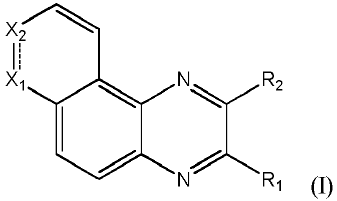 ou un sel et/ou solvate pharmaceutiquement acceptable de celui-ci, un stéréoisomère, ou un mélange de stéréoisomères en toutes proportions, en particulier un mélange d'énantiomères, et notamment un mélange racémique,
dans laquelle :
- -̅-̅-̅-̅-̅-̅ représente une liaison simple ou double, de préférence une liaison double,
- X₁ représente :
▪ NR₁ₐ lorsque -̅-̅-̅-̅-̅-̅ représente une liaison simple, et
▪ N lorsque -̅-̅-̅-̅-̅-̅ représente une liaison double,
- X₂ représente :
▪ CR₂ₐR_{2b} lorsque -̅-̅-̅-̅-̅-̅ représente une liaison simple, et
▪ CR_{2c} lorsque -̅-̅-̅-̅-̅-̅ représente une liaison double,
- R₁ et R₂ représentent chacun, indépendemment l'un de l'autre, un atome d'hydrogène ; un atome d'halogène tel qu'un atome de chlore, brome ou fluor ; une chaîne hydrocarbonée saturée ou insaturée, linéaire ou ramifiée, comportant de 1 à 10, de préférence 1 à 6 atomes de carbone ; un aryle éventuellement substitué ; ou un hétéroaryle éventuellement substitué,
- R₁ₐ et R_{2c} représentent chacun, indépendemment l'un de l'autre, un atome d'hydrogène ou un groupe (C₁-C₆)alkyle, et
- R₂ₐ et R_{2b} représentent chacun, indépendemment l'un de l'autre, un groupe (C₁-C₆)alkyle,
à l'exclusion :
• de la pyrido[3,2-f]quinoxaline,
• de la 2,3-diméthyl-pyrido[3,2-f]quinoxaline,
• de la 2,3,8-triméthyl-pyrido[3,2-f]quinoxaline, et
• de la 2,3-diphényl-pyrido[3,2-f]quinoxaline.

2. Composé selon la revendication 1, **caractérisé en ce qu'**il s'agit d'un composé de formule (Ia) suivante : 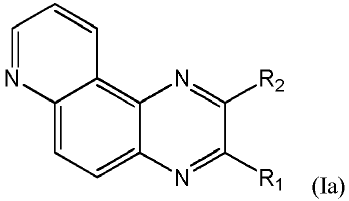 ou un sel et/ou solvate pharmaceutiquement acceptable de celui-ci, un stéréoisomère, ou un mélange de stéréoisomères en toutes proportions, en particulier un mélange d'énantiomères, et notamment un mélange racémique,
dans laquelle R₁ et R₂ sont tels que définis à la revendication 1.

3. Composé selon l'une quelconque des revendications 1 et 2, **caractérisé en ce que** R₁ et R₂ représentent chacun, indépendemment l'un de l'autre :
- un atome d'hydrogène,
- un atome d'halogène tel que le chlore, le fluor et le brome,
- un (C₁-C₆)alkyle,
- un (C₂-C₆)alkynyle, comportant avantageusement une et une seule triple liaison,
- un aryle éventuellement substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH, ou
- un hétéroaryle éventuellement substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH.

4. Composé selon l'une quelconque des revendications 1 à 3, **caractérisé en ce que** R₁ et R₂ représentent chacun, indépendemment l'un de l'autre, un atome d'hydrogène ; un groupe (C₁-C₆)alkyle ; ou un groupe aryle ou hétéroaryle éventuellement substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH.

5. Composé selon l'une quelconque des revendications 1 à 4, **caractérisé en ce que** R₁ et R₂ représentent chacun chacun, indépendemment l'un de l'autre, un atome d'hydrogène ; un groupe (C₁-C₆)alkyle ; ou un groupe phényle, naphtyle, pyridyle, quinoxalyle ou quinolyle éventuellement substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle); de préférence choisis parmi un atome d'halogène, (C₁-C₆)alkyle, (C₁-C₆)alcoxy, OH et aryle ; notamment choisis parmi (C₁-C₆)alcoxy et OH.

6. Composé selon l'une quelconque des revendications 1 à 4, **caractérisé en ce que** R₁ représente un hydrogène ou un groupe (C₁-C₆)alkyle et R₂ représente un groupe aryle ou hétéroaryle, de préférence aryle, éventuellement substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH.

7. Composé selon la revendication 6, **caractérisé en ce que** R₂ représente un groupe phényle, naphtyle, pyridyle, quinoxalyle ou quinolyle, de préférence phényle, éventuellement substitué par un ou plusieurs groupes choisis parmi un atome d'halogène, (C₁-C₆)alkyle, (C₁-C₆)alcoxy, aryle, N₃, NO₂, OH, NH₂, et -NH-((C₁-C₆)alkyle) ; de préférence choisis parmi un atome d'halogène, (C₁-C₆)alkyle, (C₁-C₆)alcoxy, OH et aryle ; notamment choisis parmi (C₁-C₆)alcoxy et OH.

8. Composé selon la revendication 1, **caractérisé en ce qu'**il est choisi parmi les composés suivants :
| | | | |
|---|---|---|---|
| **Iac** | 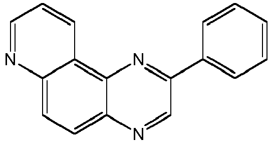 | **Iaf** | 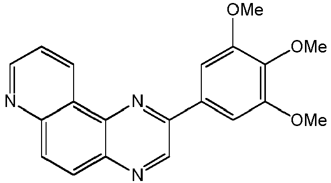 |
| **Iad** | 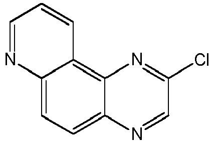 | **Iag** | 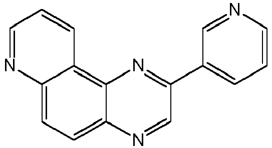 |
| **Iae** | 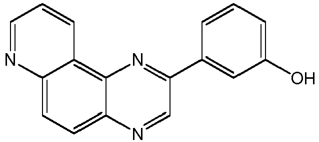 | **Iam** | 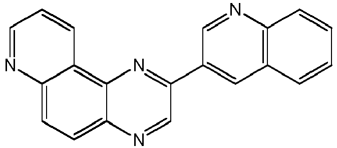 |
| **Iai** | 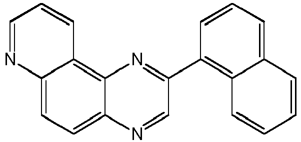 | **Iah** | 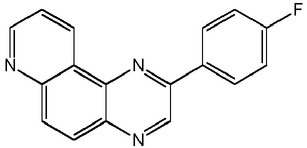 |
| **Iak** | 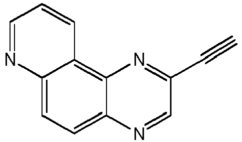 | **Iaj** | 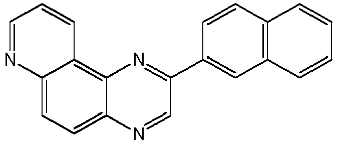 |
| **Ibl** | 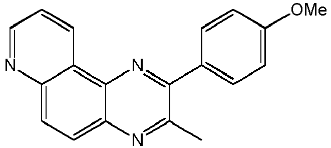 | **Ibn** | 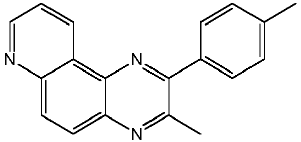 |
| **Ibo** | 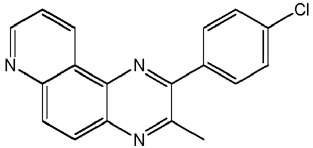 | **Ibf** | 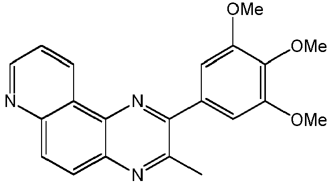 |
| **Ibh** | 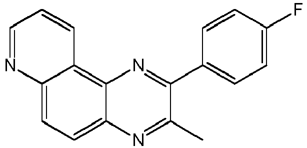 | **Ibp** | 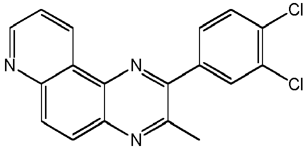 |
| **Ibj** | 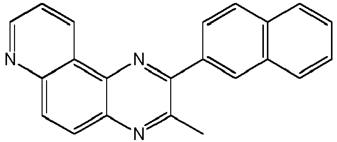 | **Ibq** | 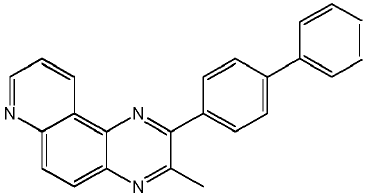 |
| **Xbc** | 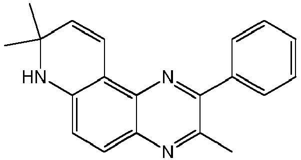 | | |
et leurs sels et/ou solvates pharmaceutiquement acceptables.

9. Composé selon l'une quelconque des revendications 1 à 8 ou pyrido[3,2-f]quinoxaline, 2,3 -diméthyl-pyrido [3,2-f]quinoxaline, 2,3,8 -triméthyl-pyrido [3,2-f]quinoxaline ou 2,3-diphényl-pyrido[3,2-f]quinoxaline, pour son utilisation en tant que médicament, notamment en tant que médicament neurotrophique ou neuroprotecteur.

10. Composé selon l'une quelconque des revendications 1 à 8 ou pyrido[3,2-f]quinoxaline, 2,3-diméthyl-pyrido[3,2-f]quinoxaline, 2,3,8-triméthyl-pyrido[3,2-f]quinoxaline ou 2,3-diphényl-pyrido[3,2-f]quinoxaline, pour son utilisation dans le traitement ou la prévention d'une maladie neurodégénérative.

11. Composé pour son utilisation selon la revendication 10, **caractérisé en ce que** la maladie neurodégénérative est la maladie d'Alzheimer, la maladie de Parkinson, la sclérose en plaques ou la sclérose latérale amyotrophique, et en particulier la maladie de Parkinson.

12. Composition pharmaceutique comprenant au moins un composé choisi parmi les composés de formule (I) selon l'une quelconque des revendications 1 à 8, la pyrido[3,2-f]quinoxaline, la 2,3-diméthyl-pyrido[3,2-f]quinoxaline, la 2,3,8-triméthyl-pyrido[3,2-f]quinoxaline et la 2,3-diphényl-pyrido[3,2-f]quinoxaline ; et un véhicule pharmaceutiquement acceptable,
à l'exclusion d'une solution de pyrido[3,2-f]quinoxaline dans l'eau.

13. Procédé de préparation d'un composé de formule (I) selon la revendication 1, de la pyrido[3,2-f]quinoxaline, de la 2,3-diméthyl-pyrido[3,2-f]quinoxaline, de la 2,3,8-triméthyl-pyrido[3,2-f]quinoxaline ou de la 2,3-diphényl-pyrido[3,2-f]quinoxaline, comprenant les étapes successives suivantes :
(a1) couplage entre une amino-quinoxaline de formule (II) suivante : 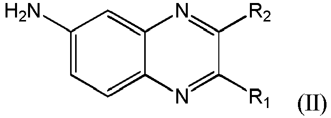 pour laquelle R₁ et R₂ sont tels que définis à la revendication 1,
avec un halogénure propargylique de formule CH≡C-CHR₂CHal ou CH≡C-CR₂ₐR_{2b}Hal dans laquelle R₂ₐ, R_{2b} et R_{2c} sont tels que définis à la revendication 1 et Hal représente un atome d'halogène tel que Cl, Br ou I, pour donner un composé de formule (IIIa) ou (IIIb) suivante : 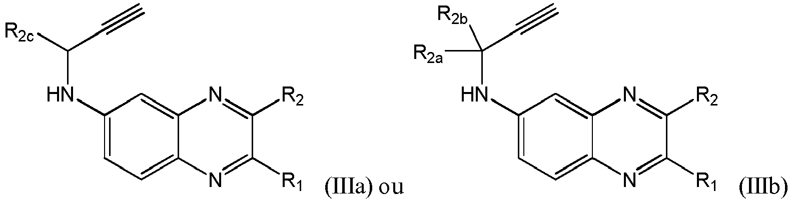 pour laquelle R₁, R₂, R₂ₐ, R_{2b} et R_{2c} sont tels que définis à la revendication 1,
(b1) cycloisomérisation du composé de formule (IIIa) ou (IIIb) obtenu à l'étape précédente et aromatisation lorsque -̅-̅-̅-̅-̅-̅ représente une liaison double pour donner un composé de formule (I), et
(c1) éventuellement salification et/ou solvatation du composé de formule (I) obtenu à l'étape précédente pour donner un sel et/ou solvate pharmaceutiquement acceptable du composé de formule (I).

14. Procédé de préparation d'un composé de formule (I) selon la revendication 1 pour lequel au moins l'un de R₁ et R₂ représente une chaîne hydrocarbonée saturée ou insaturée, linéaire ou ramifiée, comportant de 1 à 10 atomes de carbone ; un aryle éventuellement substitué ; ou un hétéroaryle éventuellement substitué ; de la 2,3-diméthyl-pyrido[3,2-f]quinoxaline, de la 2,3,8-triméthyl-pyrido[3,2-f]quinoxaline ou de la 2,3-diphényl-pyrido[3,2-f]quinoxaline, comprenant les étapes successives suivantes :
(a2) couplage d'un composé de formule (I) selon la revendication 1 pour lequel au moins l'un de R₁ et R₂ représente un atome d'halogène, tel que Cl, Br ou I,
avec un dérivé d'acide boronique de formule R₃-B(R₄)₂ ou R₃-BF₃⁻K⁺ pour lequel R₃ représente une chaîne hydrocarbonée saturée ou insaturée, linéaire ou ramifiée, comportant de 1 à 10 atomes de carbone ; un aryle éventuellement substitué ; ou un hétéroaryle éventuellement substitué, et R₄ représente un groupe (C₁-C₆)alkyle, OH ou (C₁-C₆)alcoxy,
ou avec un dérivé zincique de formule R₃-Zn-Hal pour lequel R₃ est tel que défini ci-dessus et Hal représente un atome d'halogène tel que Cl, Br ou I,
ou avec un dérivé stannane de formule R₃-SnA₁A₂A₃ pour lequel R₃ est tel que défini ci-dessus et A₁, A₂ et A₃, identiques ou différents, représentent chacun un groupe (C₁-C₆)alkyle,
ou avec un dérivé magnésien de formule R₃-Mg-Hal pour lequel R₃ et Hal sont tels que définis ci-dessus,
ou avec un derivé silicié de formule R₃-SiMe₂OH, R₃-SiF₃ ou R₃-Si(OA₁)(OA₂)(OA₃) pour lequel R₃, A₁, A₂ et A₃ sont tels que définis ci-dessus, ou avec un alcyne de formule R'-C≡CH pour lequel R' représente un groupe protecteur ou une chaîne hydrocarbonée saturée ou insaturée, linéaire ou ramifiée, comportant de 1 à 8 atomes de carbone,
(b2) lorsque l'étape (a2) a été réalisée avec un alcyne de formule R'-C=CH pour lequel R' représente un groupe protecteur, déprotection du groupe protecteur de la fonction alcyne, et
(c2) éventuellement salification et/ou solvatation du composé de formule (I) obtenu à l'étape précédente pour donner un sel et/ou solvate pharmaceutiquement acceptable du composé de formule (I).

## Patentansprüche

1. Verbindung der folgenden Formel (I): 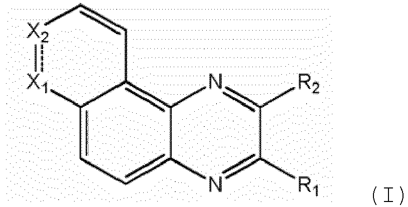 oder ein pharmazeutisch verträgliches Salz und/oder Solvat davon, ein Stereoisomer, oder eine Mischung von Stereoisomeren in allen Verhältnissen, insbesondere eine Mischung von Enantiomeren, und vor allem eine racemische Mischung,
wobei:
- -̅-̅-̅-̅-̅-̅ für eine Einfach- oder Doppelbindung, vorzugsweise eine Doppelbindung steht,
- X₁ steht für:
▪ NR₁ₐ, wenn -̅-̅-̅-̅-̅-̅ für eine Einfachbindung steht, und
▪ N, wenn -̅-̅-̅-̅-̅-̅ für eine Doppelbindung steht,
- X₂ steht für:
▪ CR₂ₐR_{2b}, wenn -̅-̅-̅-̅-̅-̅ für eine Einfachbindung steht, und
▪ CR_{2c}, wenn -̅-̅-̅-̅-̅-̅ für eine Doppelbindung steht,
- R₁ und R₂ jeweils unabhängig voneinander für ein Wasserstoffatom; ein Halogenatom wie etwa ein Chlor-, Brom- oder Fluoratom; eine gesättigte oder ungesättigte, lineare oder verzweigte Kohlenwasserstoffkette, die 1 bis 10, vorzugsweise 1 bis 6 Kohlenstoffatome umfasst; ein gegebenenfalls substituiertes Aryl; oder ein gegebenenfalls substituiertes Heteroaryl stehen,
- R₁ₐ und R_{2c} jeweils unabhängig voneinander für ein Wasserstoffatom oder eine (C₁-C₆)-Alkylgruppe stehen, und
- R₂ₐ und R_{2b} jeweils unabhängig voneinander für eine (C₁-C₆)-Alkylgruppe stehen,
mit Ausnahme:
-- von Pyrido[3,2-f]chinoxalin,
-- von 2,3-Dimethylpyrido[3,2-f]chinoxalin,
-- von 2,3,8-Trimethylpyrido[3,2-f]chinoxalin, und
-- von 2,3-Diphenylpyrido[3,2-f]chinoxalin.

2. Verbindung nach Anspruch 1, **dadurch gekennzeichnet, dass** es sich um eine Verbindung der folgenden Formel (Ia): 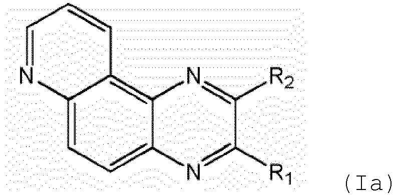 oder ein pharmazeutisch verträgliches Salz und/oder Solvat davon, ein Stereoisomer, oder eine Mischung von Stereoisomeren in allen Verhältnissen, insbesondere eine Mischung von Enantiomeren, und vor allem eine racemische Mischung handelt,
wobei R₁ und R₂ wie in Anspruch 1 definiert sind.

3. Verbindung nach einem der Ansprüche 1 und 2, **dadurch gekennzeichnet, dass** R₁ und R₂ jeweils unabhängig voneinander stehen für:
- ein Wasserstoffatom,
- ein Halogenatom wie etwa Chlor, Fluor und Brom,
- ein (C₁-C₆)-Alkyl,
- ein (C₂-C₆)-Alkinyl, das vorteilhafterweise eine, und nur eine Dreifachbindung umfasst,
- ein Aryl, das gegebenenfalls durch eine oder mehrere Gruppen substituiert ist, ausgewählt aus einem Halogenatom, (C₁-C₆)-Alkyl, (C₁-C₆)-Alkoxy, Aryl, N₃, NO₂, OH, NH₂, und -NH-((C₁-C₆)-Alkyl) ; vorzugsweise ausgewählt aus einem Halogenatom, (C₁-C₆)-Alkyl, (C₁-C₆)-Alkoxy, OH und Aryl; vor allem ausgewählt aus (C₁-C₆)-Alkoxy und OH, oder
- ein Heteroaryl, das gegebenenfalls durch eine oder mehrere Gruppen substituiert ist, ausgewählt aus einem Halogenatom, (C₁-C₆)-Alkyl, (C₁-C₆)-Alkoxy, Aryl, N₃, NO₂, OH, NH₂, und -NH- ((C₁-C₆)-Alkyl) ; vorzugsweise ausgewählt aus einem Halogenatom, (C₁-C₆)-Alkyl, (C₁-C₆)-Alkoxy, OH und Aryl; vor allem ausgewählt aus (C₁-C₆)-Alkoxy und OH.

4. Verbindung nach einem der Ansprüche 1 bis 3, **dadurch gekennzeichnet, dass** R₁ und R₂ jeweils unabhängig voneinander für ein Wasserstoffatom; eine (C₁-C₆)-Alkylgruppe; oder eine Aryl- oder Heteroarylgruppe stehen, die gegebenenfalls durch eine oder mehrere Gruppen substituiert ist, ausgewählt aus einem Halogenatom, (C₁-C₆)-Alkyl, (C₁-C₆)-Alkoxy, Aryl, N₃, NO₂, OH, NH₂, und -NH-((C₁-C₆)-Alkyl); vorzugsweise ausgewählt aus einem Halogenatom, (C₁-C₆)-Alkyl, (C₁-C₆)-Alkoxy, OH und Aryl; vor allem ausgewählt aus (C₁-C₆)-Alkoxy und OH.

5. Verbindung nach einem der Ansprüche 1 bis 4, **dadurch gekennzeichnet, dass** R₁ und R₂ jeweils unabhängig voneinander für ein Wasserstoffatom; eine (C₁-C₆)-Alkylgruppe; oder eine Phenyl-, Naphthyl-, Pyridyl-, Chinoxalyl- oder Chinolylgruppe stehen, die gegebenenfalls durch eine oder mehrere Gruppen substituiert ist, ausgewählt aus einem Halogenatom, (C₁-C₆)-Alkyl, (C₁-C₆)-Alkoxy, Aryl, N₃, NO₂, OH, NH₂, und -NH-((C₁-C₆)-Alkyl); vorzugsweise ausgewählt aus einem Halogenatom, (C₁-C₆)-Alkyl, (C₁-C₆)-Alkoxy, OH und Aryl; vor allem ausgewählt aus (C₁-C₆)-Alkoxy und OH.

6. Verbindung nach einem der Ansprüche 1 bis 4, **dadurch gekennzeichnet, dass** R₁ für einen Wasserstoff oder eine (C₁-C₆)-Alkylgruppe steht, und R₂ für eine Aryl- oder Heteroaryl-, vorzugsweise Arylgruppe steht, die gegebenenfalls durch eine oder mehrere Gruppen substituiert ist, ausgewählt aus einem Halogenatom, (C₁-C₆)-Alkyl, (C₁-C₆)-Alkoxy, Aryl, N₃, NO₂, OH, NH₂, und -NH-((C₁-C₆)-Alkyl); vorzugsweise ausgewählt aus einem Halogenatom, (C₁-C₆)-Alkyl, (C₁-C₆)-Alkoxy, OH und Aryl; vor allem ausgewählt aus (C₁-C₆)-Alkoxy und OH.

7. Verbindung nach Anspruch 6, **dadurch gekennzeichnet, dass** R₂ für eine Phenyl-, Naphthyl-, Pyridyl-, Chinoxalyl- oder Chinolyl-, vorzugsweise Phenylgruppe steht, die gegebenenfalls durch eine oder mehrere Gruppen substituiert ist, ausgewählt aus einem Halogenatom, (C₁-C₆)-Alkyl, (C₁-C₆)-Alkoxy, Aryl, N₃, NO₂, OH, NH₂, und -NH-((C₁-C₆)-Alkyl); vorzugsweise ausgewählt aus einem Halogenatom, (C₁-C₆)-Alkyl, (C₁-C₆)-Alkoxy, OH und Aryl; vor allem ausgewählt aus (C₁-C₆)-Alkoxy und OH.

8. Verbindung nach Anspruch 1, **dadurch gekennzeichnet, dass** sie aus den folgenden Verbindungen:
| | | | |
|---|---|---|---|
| **Iac** | 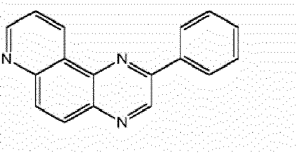 | **Iaf** | 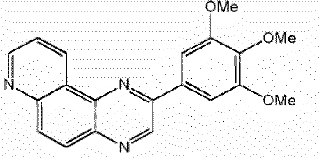 |
| **Iad** | 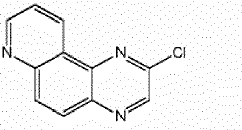 | **Iag** | 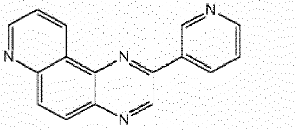 |
| **Iae** | 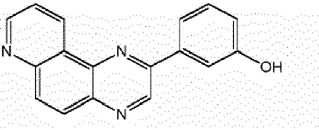 | **Iam** | 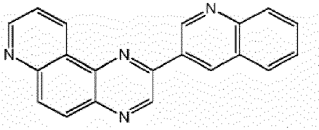 |
| **Iai** | 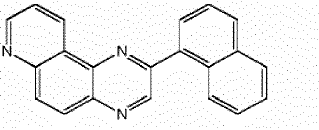 | **Iah** | 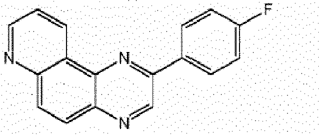 |
| **Iak** | 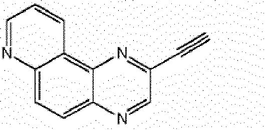 | **Iaj** | 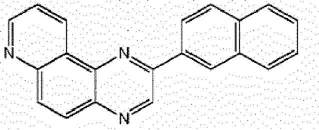 |
| **Ibl** | 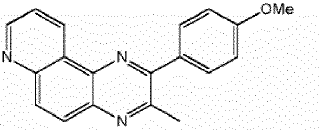 | **Ibn** | 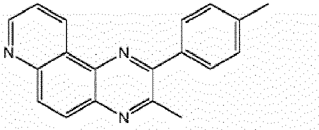 |
| **Ibo** | 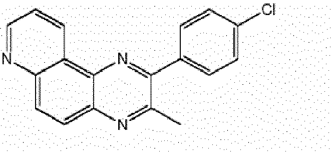 | **Ibf** | 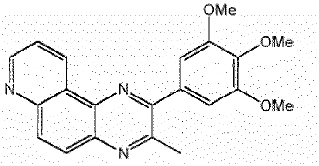 |
| **Ibh** | 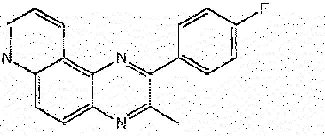 | **Ibp** | 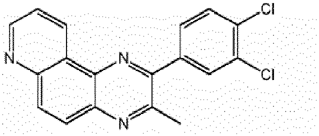 |
| **Ibj** | 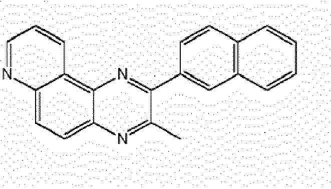 | **Ibq** | 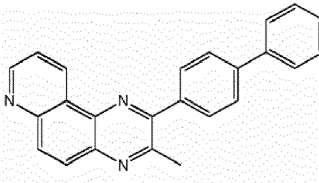 |
| **Xbc** | 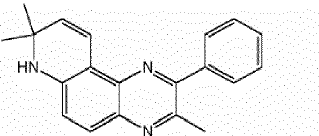 | | |
und deren pharmazeutisch verträglichen Salzen und/oder Solvaten ausgewählt ist.

9. Verbindung nach einem der Ansprüche 1 bis 8 oder Pyrido[3,2-f]chinoxalin, 2,3-Dimethylpyrido[3,2-f]chinoxalin, 2,3,8-Trimethylpyrido[3,2-f]chinoxalin oder 2,3-Diphenylpyrido[3,2-f]chinoxalin zur deren/dessen Verwendung als Arzneimittel, insbesondere als neurotropes oder neuroprotektives Arzneimittel.

10. Verbindung nach einem der Ansprüche 1 bis 8 oder Pyrido[3,2-f]chinoxalin, 2,3-Dimethylpyrido[3,2-f]chinoxalin, 2,3,8-Trimethylpyrido[3,2-f]chinoxalin oder 2,3-Diphenylpyrido[3,2-f]chinoxalin zur deren/dessen Verwendung in der Behandlung oder der Vorbeugung einer neurodegenerativen Erkrankung.

11. Verbindung zu deren Verwendung nach Anspruch 10, **dadurch gekennzeichnet, dass** die neurodegenerative Erkrankung die Alzheimer-Erkrankung, die Parkinson-Erkrankung, die multiple Sklerose oder die amyotrophe Lateralsklerose, und insbesondere die Parkinson-Erkrankung ist.

12. Pharmazeutische Zusammensetzung, die mindestens eine Verbindung, ausgewählt aus den Verbindungen der Formel (I) nach einem der Ansprüche 1 bis 8, Pyrido[3,2-f]chinoxalin, 2,3-Dimethylpyrido[3,2-f]chinoxalin, 2,3,8-Trimethylpyrido[3,2-f]chinoxalin und 2,3-Diphenylpyrido[3,2-f]chinoxalin; und ein pharmazeutisch verträgliches Vehikel umfasst,
mit Ausnahme einer Lösung von Pyrido[3,2-f]chinoxalin in Wasser.

13. Verfahren zur Herstellung einer Verbindung der Formel (I) nach Anspruch 1, von Pyrido[3,2-f]chinoxalin, von 2,3-Dimethylpyrido[3,2-f]chinoxalin, von 2,3,8-Trimethylpyrido[3,2-f]chinoxalin oder von 2,3-Diphenylpyrido[3,2-f]chinoxalin, das die folgenden aufeinanderfolgenden Schritte umfasst:
(a1) Koppeln zwischen einem Aminochinoxalin der folgenden Formel (II): 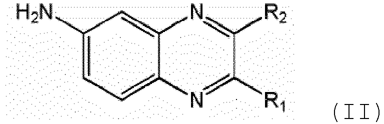 wobei R₁ und R₂ wie in Anspruch 1 definiert sind,
mit einem Propargylhalogenid der Formel CH≡C-CHR_{2c}Hal oder CH≡C-CR₂ₐR_{2b}Hal, wobei R₂ₐ, R_{2b} und R_{2c} wie in Anspruch 1 definiert sind und Hal für ein Halogenatom wie etwa Cl, Br oder I steht,
um eine Verbindung der folgenden Formel (IIIA) oder (IIIb): 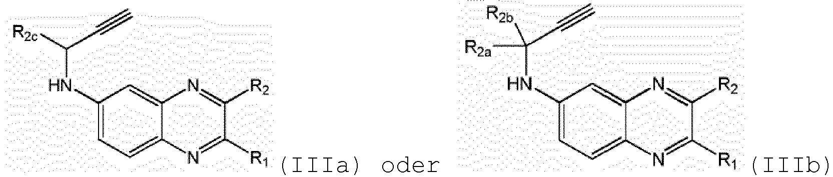 zu bilden, wobei R₁, R₂, R₂ₐ, R_{2b} und R_{2c} wie in Anspruch 1 definiert sind,
(b1) Cycloisomerisieren der im vorhergehenden Schritt erhaltenen Verbindung der Formel (IIIa) oder (IIIb) und Aromatisieren, wenn -̅-̅-̅-̅-̅-̅ für eine Doppelbindung steht, um eine Verbindung der Formel (I) zu bilden, und
(c1) gegebenenfalls Versalzen und/oder Solvatisieren der im vorhergehenden Schritt erhaltenen Verbindung der Formel (I), um ein pharmazeutisch verträgliches Salz und/oder Solvat der Verbindung der Formel (I) zu bilden.

14. Verfahren zur Herstellung einer Verbindung der Formel (I) nach Anspruch 1, wobei mindestens eines aus R₁ und R₂ für eine gesättigte oder ungesättigte, lineare oder verzweigte Kohlenwasserstoffkette, die 1 bis 10 Kohlenstoffatome umfasst; ein gegebenenfalls substituiertes Aryl; oder ein gegebenenfalls substituiertes Heteroaryl steht; von 2,3-Dimethylpyrido[3,2-f]chinoxalin, von 2,3,8-Trimethylpyrido[3,2-f]chinoxalin oder von 2,3-Diphenylpyrido[3,2-f]chinoxalin, das die folgenden aufeinanderfolgenden Schritte umfasst:
(a2) Koppeln einer Verbindung der Formel (I) nach Anspruch 1, wobei mindestens eines aus R₁ und R₂ für ein Halogenatom wie etwa Cl, Br oder I steht,
mit einem Boronsäurederivat der Formel R₃-B(R₄)₂ oder R₃-BF₃⁻K⁺, wobei R₃ für eine gesättigte oder ungesättigte, lineare oder verzweigte Kohlenwasserstoffkette, die 1 bis 10 Kohlenstoffatome umfasst; ein gegebenenfalls substituiertes Aryl; oder ein gegebenenfalls substituiertes Heteroaryl steht, und R₄ für eine (C₁-C₆)-Alkyl-, OH- oder (C₁-C₆)-Alkoxygruppe steht,
oder mit einem Zinkderivat der Formel R₃-Zn-Hal, wobei R₃ wie oben definiert ist und Hal für ein Halogenatom wie etwa Cl, Br oder I steht,
oder mit einem Stannanderivat der Formel R₃-SnA₁A₂A₃, wobei R₃ wie oben definiert ist und A₁, A₂ und A₃, identisch oder verschieden, jeweils für eine (C₁-C₆)-Alkylgruppe stehen,
oder mit einem Magnesiumderivat der Formel R₃-Mg-Hal, wobei R₃ und Hal wie oben definiert sind,
oder mit einem Siliciumderivat der Formel R₃-SiMe₂OH, R₃-SiF₃ oder R₃-Si(OA₁)(OA₂)(OA₃), wobei R₃, A₁, A₂ und A₃ wie oben definiert sind,
oder mit einem Alkin der Formel R'-C≡CH, wobei R' für eine Schutzgruppe oder eine gesättigte oder ungesättigte, lineare oder verzweigte Kohlenwasserstoffkette steht, die 1 bis 8 Kohlenstoffatome umfasst,
(b2) wenn der Schritt (a2) mit einem Alkin der Formel R'-C≡CH ausgeführt wurde, wobei R' für eine Schutzgruppe steht, Entschützen der Schutzgruppe der Alkinfunktion, und
(c2) gegebenenfalls Versalzen und/oder Solvatisieren der im vorhergehenden Schritt erhaltenen Verbindung der Formel (I), um ein pharmazeutisch verträgliches Salz und/oder Solvat der Verbindung der Formel (I) zu bilden.

## Claims

1. A compound of following formula (I): 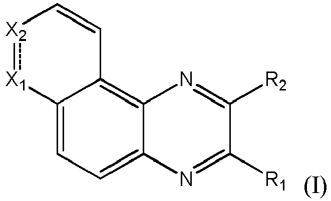 or a pharmaceutically acceptable salt and/or solvate thereof, a stereoisomer, or a mixture of stereoisomers in any proportions, in particular a mixture of enantiomers, and particularly a racemic mixture,
wherein:
- -̅-̅-̅-̅-̅-̅ is a single or double bond, preferably a double bond,
- X₁ is:
▪ NR₁ₐ when -̅-̅-̅-̅-̅-̅ is a single bond, and
▪ N when -̅-̅-̅-̅-̅-̅ is a double bond,
- X₂ is:
▪ CR₂ₐR_{2b} when -̅-̅-̅-̅-̅-̅ is a single bond, and
▪ CR_{2c} when -̅-̅-̅-̅-̅-̅ is a double bond,
- R₁ and R₂ are each independently a hydrogen atom; a halogen atom such as a chlorine, bromine or fluorine atom; a linear or branched, saturated or unsaturated hydrocarbon chain having from 1 to 10, preferably 1 to 6 carbon atoms; an optionally substituted aryl; or an optionally substituted heteroaryl,
- R₁ₐ and R_{2c} are each independently a hydrogen atom or a (C₁-C₆)alkyl group, and
- R₂ₐ and R_{2b} are each independently a (C₁-C₆)alkyl group,
with the exception of:
• pyrido[3,2-f]quinoxaline,
• 2,3-dimethyl-pyrido[3,2-f]quinoxaline,
• 2,3,8-trimethyl-pyrido[3,2-f]quinoxaline, and
• 2,3-diphenyl-pyrido[3,2-f]quinoxaline.

2. The compound according to claim 1, **characterized in that** it is a compound of following formula (Ia): 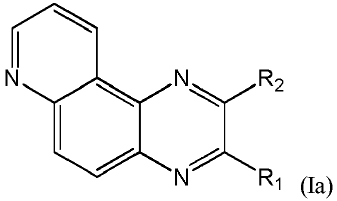 or a pharmaceutically acceptable salt and/or solvate thereof, a stereoisomer, or a mixture of stereoisomers in any proportions, in particular a mixture of enantiomers, and particularly a racemic mixture,
wherein R₁ and R₂ are as defined in claim 1.

3. The compound according to any one of claims 1 and 2, **characterized in that** R₁ and R₂ are each independently:
- a hydrogen atom,
- a halogen atom such as chlorine, fluorine and bromine,
- a (C₁-C₆)alkyl,
- a (C₂-C₆)alkynyl, advantageously having one and only one triple bond,
- an aryl optionally substituted by one or more groups selected from a halogen atom, (C₁-C₆)alkyl, (C₁-C₆)alkoxy, aryl, N₃, NO₂, OH, NH₂, and -NH-((C₁-C₆)alkyl); preferably selected from a halogen atom, (C₁-C₆)alkyl, (C₁-C₆)alkoxy, OH and aryl; particularly selected from (C₁-C₆)alkoxy and OH, or
- a heteroaryl optionally substituted by one or more groups selected from a halogen atom, (C₁-C₆)alkyl, (C₁-C₆)alkoxy, aryl, N₃, NO₂, OH, NH₂, and -NH-((C₁-C₆)alkyl); preferably selected from a halogen atom, (C₁-C₆)alkyl, (C₁-C₆)alkoxy, OH and aryl; particularly selected from (C₁-C₆)alkoxy and OH.

4. The compound according to any one of claims 1 to 3, **characterized in that** R₁ and R₂ are each independently a hydrogen atom; a (C₁-C₆)alkyl group; or an aryl or heteroaryl group optionally substituted by one or more groups selected from a halogen atom, (C₁-C₆)alkyl, (C₁-C₆)alkoxy, aryl, N₃, NO₂, OH, NH₂, and -NH-((C₁-C₆)alkyl); preferably selected from a halogen atom, (C₁-C₆)alkyl, (C₁-C₆)alkoxy, OH and aryl; particularly selected from (C₁-C₆)alkoxy and OH.

5. The compound according to any one of claims 1 to 4, **characterized in that** R₁ and R₂ are each independently a hydrogen atom; a (C₁-C₆)alkyl group; or a phenyl, naphthyl, pyridyl, quinoxalyl or quinolyl group optionally substituted by one or more groups selected from a halogen atom, (C₁-C₆)alkyl, (C₁-C₆)alkoxy, aryl, N₃, NO₂, OH, NH₂, and -NH-((C₁-C₆)alkyl); preferably selected from a halogen atom, (C₁-C₆)alkyl, (C₁-C₆)alkoxy, OH and aryl; particularly selected from (C₁-C₆)alkoxy and OH.

6. The compound according to any one of claims 1 to 4, **characterized in that** R₁ is a hydrogen or a (C₁-C₆)alkyl group and R₂ is an aryl or heteroaryl group, preferably aryl, optionally substituted by one or more groups selected from a halogen atom, (C₁-C₆)alkyl, (C₁-C₆)alkoxy, aryl, N₃, NO₂, OH, NH₂, and -NH-((C₁-C₆)alkyl); preferably selected from a halogen atom, (C₁-C₆)alkyl, (C₁-C₆)alkoxy, OH and aryl; particularly selected from (C₁-C₆)alkoxy and OH.

7. The compound according to claim 6, **characterized in that** R₂ is a phenyl, naphthyl, pyridyl, quinoxalyl or quinolyl group, preferably phenyl, optionally substituted by one or more groups selected from a halogen atom, (C₁-C₆)alkyl, (C₁-C₆)alkoxy, aryl, N₃, NO₂, OH, NH₂, and -NH-((C₁-C₆)alkyl); preferably selected from a halogen atom, (C₁-C₆)alkyl, (C₁-C₆)alkoxy, OH and aryl; particularly selected from (C₁-C₆)alkoxy and OH.

8. The compound according to claim 1, **characterized in that** it is selected from the following compounds:
| | | | |
|---|---|---|---|
| **Iac** | 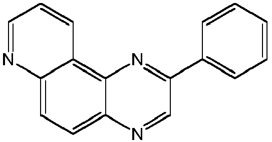 | **Iaf** | 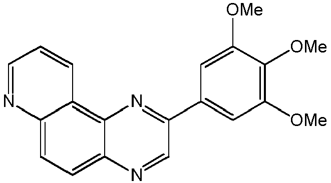 |
| **Iad** | 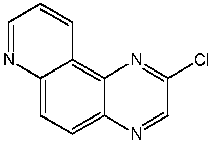 | **Iag** | 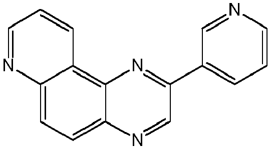 |
| **Iae** | 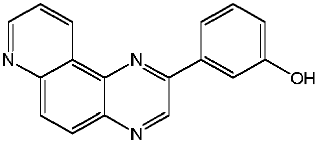 | **Iam** | 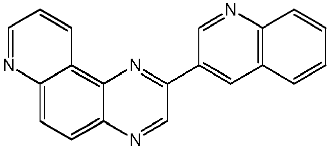 |
| **Iai** | 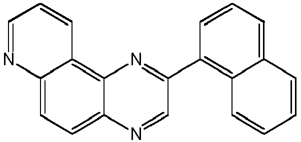 | **Iah** | 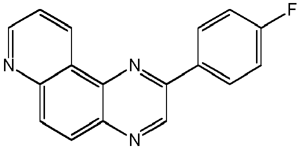 |
| **Iak** | 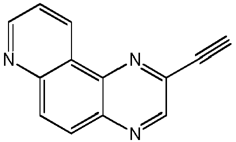 | **Iaj** | 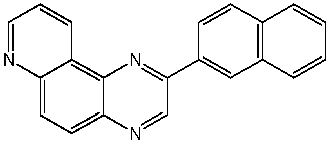 |
| **Ibl** | 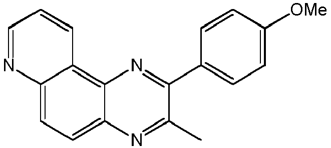 | **Ibn** | 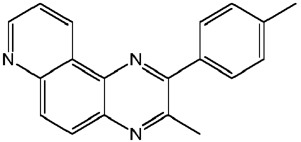 |
| **Ibo** | 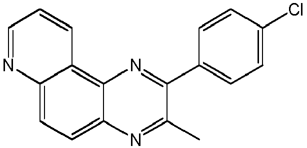 | **Ibf** | 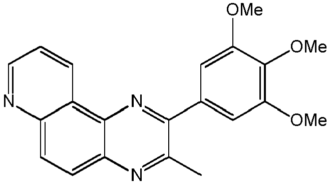 |
| **Ibh** | 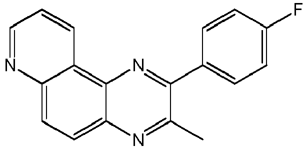 | **Ibp** | 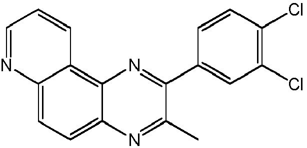 |
| **Ibj** | 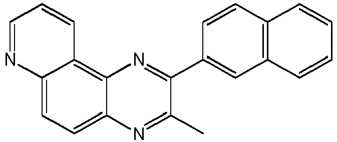 | **Ibq** | 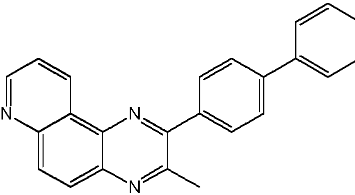 |
| **Xbc** | 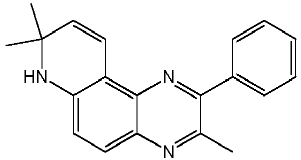 | | |
and the pharmaceutically acceptable salts and/or solvates thereof.

9. The compound according to any one of claims 1 to 8 or pyrido[3,2-f]quinoxaline, 2,3-dimethyl-pyrido[3,2-f]quinoxaline, 2,3,8-trimethyl-pyrido[3,2-f]quinoxaline or 2,3-diphenyl-pyrido[3,2-f]quinoxaline, for use as a medicinal product, particularly as a neurotrophic or neuroprotective medicinal product.

10. The compound according to any one of claims 1 to 8 or pyrido[3,2-f]quinoxaline, 2,3-dimethyl-pyrido[3,2-f]quinoxaline, 2,3,8-trimethyl-pyrido[3,2-f]quinoxaline or 2,3-diphenyl-pyrido[3,2-f]quinoxaline, for use in the treatment or prevention of a neurodegenerative disease.

11. The compound for use according to claim 10, **characterized in that** the neurodegenerative disease is Alzheimer's disease, Parkinson's disease, multiple sclerosis or amyotrophic lateral sclerosis, and in particular Parkinson's disease.

12. A pharmaceutical composition comprising at least one compound selected from the compounds of formula (I) according to any one of claims 1 to 8, pyrido[3,2-f]quinoxaline, 2,3-dimethyl-pyrido[3,2-f]quinoxaline, 2,3,8-trimethyl-pyrido[3,2-f]quinoxaline and 2,3-diphenyl-pyrido[3,2-f]quinoxaline; and a pharmaceutically acceptable vehicle,
with the exception of a solution of pyrido[3,2-f]quinoxaline in water.

13. A process for preparing a compound of formula (I) according to claim 1, pyrido[3,2-f]quinoxaline, 2,3-dimethyl-pyrido[3,2-f]quinoxaline, 2,3, 8-trimethyl-pyrido[3,2-f]quinoxaline or 2,3-diphenyl-pyrido[3,2-f]quinoxaline, comprising the following successive steps:
(a1) coupling between an amino-quinoxaline of following formula (II): 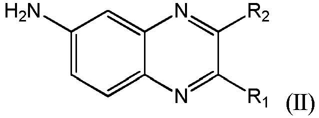 wherein R₁ and R₂ are as defined in claim 1,
with a propargyl halide of formula CH≡C-CHR_{2c}Hal or CH≡C-CR₂ₐR_{2b}Hal wherein R₂ₐ, R_{2b} and R_{2c} are as defined in claim 1 and Hal is a halogen atom such as Cl, Br or I,
to give a compound of following formula (IIIa) or (IIIb): 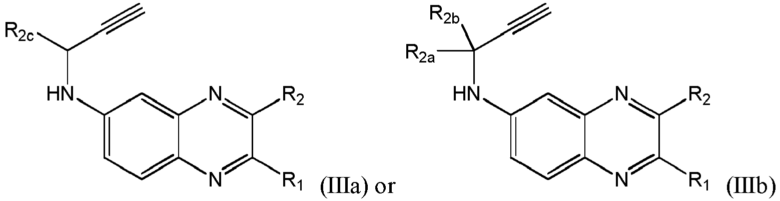 wherein R₁, R₂, R₂ₐ, R_{2b} and R_{2c} are as defined in claim 1,
(b1) cycloisomerization of the compound of formula (IIIa) or (IIIb) obtained in the preceding step and aromatization when -̅-̅-̅-̅-̅-̅ is a double bond to give a compound of formula (I), and
(c1) optionally salification and/or solvation of the compound of formula (I) obtained in the preceding step to give a pharmaceutically acceptable salt and/or solvate of the compound of formula (I).

14. A process for preparing a compound of formula (I) according to claim 1 wherein at least one of R₁ and R₂ is a linear or branched, saturated or unsaturated hydrocarbon chain having from 1 to 10 carbon atoms; an optionally substituted aryl; or an optionally substituted heteroaryl; 2,3-dimethyl-pyrido[3,2-f]quinoxaline, 2,3,8-trimethyl-pyrido[3,2-f]quinoxaline or 2,3-diphenyl-pyrido[3,2-f]quinoxaline, comprising the following successive steps:
(a2) coupling of a compound of formula (I) according to claim 1 wherein at least one of R₁ and R₂ is a halogen atom, such as Cl, Br or I,
with a boronic acid derivative of formula R₃-B(R₄)₂ or R₃-BF₃⁻K⁺ wherein R₃ is a linear or branched, saturated or unsaturated hydrocarbon chain having from 1 to 10 carbon atoms; an optionally substituted aryl; or an optionally substituted heteroaryl, and R₄ is a (C₁-C₆)alkyl, OH or (C₁-C₆)alkoxy group,
or with a zinc derivative of formula R₃-Zn-Hal wherein R₃ is as defined above and Hal is a halogen atom such as Cl, Br or I,
or with a stannane derivative of formula R₃-SnA₁A₂A₃ wherein R₃ is as defined above and A₁, A₂ and A₃, which can be identical or different, are each a (C₁-C₆)alkyl group,
or with a magnesium derivative of formula R₃-Mg-Hal wherein R₃ and Hal are as defined above,
or with a silicon derivative of formula R₃-SiMe₂OH, R₃-SiF₃ or R₃-Si(OA₁)(OA₂)(OA₃) wherein R₃, A₁, A₂ and A₃ are as defined above,
or with an alkyne of formula R'-C=CH wherein R' is a protecting group or a linear or branched, saturated or unsaturated hydrocarbon chain having from 1 to 8 carbon atoms,
(b2) when step (a2) was performed with an alkyne of formula R'-C≡CH wherein R' is a protecting group, deprotection of the protecting group of the alkyne function, and
(c2) optionally salification and/or solvation of the compound of formula (I) obtained in the preceding step to give a pharmaceutically acceptable salt and/or solvate of the compound of formula (I).
